# EUROPEAN PATENT APPLICATION

(11) **EP 4 800 800 A1**
(43) Date of publication of application: **02.09.2026**
(21) Application number: 24882550.7
(22) Date of filing: 08.04.2024
(51) Int. Cl.: H01M 10/42, H01M 10/0585, H01M 10/052, H01M 10/0525

(54) **ADDITIVE FOR LITHIUM RECHARGEABLE BATTERY AND LITHIUM RECHARGEABLE BATTERY INCLUDING SAME**

(30) Priority: 24.10.2023 KR 20230143196
(71) Applicant: SAMSUNG SDI CO., LTD., Yongin-si, Gyeonggi-do 17084 (KR); Sogang University Research Business Development Foundation, Seoul 04107 (KR)
(72) Inventor: KANG, Inyeong, Yongin-si, Gyeonggi-do 17084 (KR); CHO, Wonseok, Yongin-si, Gyeonggi-do 17084 (KR); CHOE, Yun Jeong, Yongin-si, Gyeonggi-do 17084 (KR); MOON, Bongjin, Goyang-si Gyeonggi-do 10416 (KR); KIM, Minyoung, Seoul 04209 (KR); JEON, Hyelin, Hwaseong-si, Gyeonggi-do 18423 (KR)
(74) Representative: Michalski Hüttermann & Partner mbB
(86) International application number: PCT/KR2024/004644
(87) International publication number: WO 2025/089518

(57) **Abstract**

Provided are an additive for a rechargeable lithium battery and a rechargeable lithium battery including the same, the additive being represented by Chemical Formula 1:

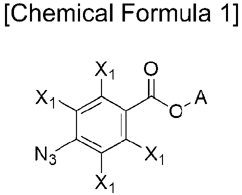

Each substituent is the same as defined in the specification.

## Description

### [Technical Field]

An additive for a rechargeable lithium battery and a rechargeable lithium battery including the same are disclosed.

### [Background Art]

A rechargeable lithium battery may be recharged and has three or more times as high energy density per unit weight as a conventional lead storage battery, nickel-cadmium battery, nickel hydrogen battery, nickel zinc battery and the like. It may be also charged at a high rate and thus, is commercially manufactured for a laptop, a cell phone, an electric tool, an electric bike, and the like, and researches on improvement of additional energy density have been actively made.

Commercially available rechargeable lithium batteries are lithium-ion batteries that use an electrolyte solution including a flammable organic solvent, and have safety issues such as explosion or fire when problems such as collision or penetration occur. Accordingly, an all-solid-state battery using a solid electrolyte instead of an electrolyte solution has been proposed. Among rechargeable lithium batteries, an all-solid-state battery refers to a battery in which all materials are made of solid, especially a battery that uses a solid electrolyte.

### [Disclosure]

### [Technical Problem]

### [Technical Solution]

An embodiment provides an additive for a rechargeable lithium battery that can suppress ignition due to abnormal heat generation, regardless of the type of the rechargeable lithium battery.

Another embodiment provides an additive for a rechargeable lithium battery represented by Chemical Formula 1:

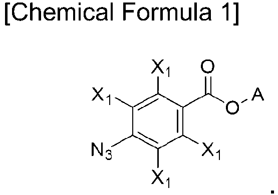

The additive for a rechargeable lithium battery according to an embodiment can be applied to at least one of the components of a rechargeable lithium battery, regardless of the type of the rechargeable lithium battery, and can suppress an ignition phenomenon due to abnormal heat generation.

### [Description of Drawings]

FIGS. 1 and 2 are cross-sectional views schematically showing an all-solid-state battery according to an embodiment.
FIG. 3 is a TGA thermal analysis diagram of the additive (compound represented by Chemical Formula 1-1a) of Synthesis Example 1.
FIG. 4 is a TGA thermal analysis diagram of the additive (compound represented by Formula 1-1b) of Synthesis Example 2.
FIG. 5 is a TGA thermal analysis diagram of the additive (compound represented by Chemical Formula 1-2a) of Synthesis Example 3.
FIG. 6 is a TGA thermal analysis diagram of the additive (compound represented by Chemical Formula 1-3a) of Synthesis Example 4.
FIG. 7 is a DSC analysis diagram of the all-solid-state battery cell of Example 1 to which the additive (compound represented by Chemical Formula 1-1a) of Synthesis Example 1 is applied.
FIG. 8 is a DSC analysis diagram of the all-solid-state battery cell of Example 1 to which the additive of Synthesis Example 2 (compound represented by Chemical Formula 1-1b) is applied.
FIG. 9 is a DSC analysis diagram of the all-solid-state battery cell of Example 1 to which the additive (compound represented by Chemical Formula 1-2a) of Synthesis Example 3 is applied.
FIG. 10 is a DSC analysis diagram of the all-solid-state battery cell of Example 1 to which the additive (compound represented by Chemical Formula 1-3a) of Synthesis Example 4 is applied.
FIG. 11 is a DSC analysis diagram of the all-solid-state battery cell of Example 1 to which the additive of Synthesis Example 5 (compound represented by Chemical Formula 1-4a) is applied.

### [Best Mode]

Hereinafter, embodiments will be described in detail so that those of ordinary skill in the art can easily implement them. However, this disclosure may be embodied in many different forms and is not construed as limited to the example embodiments set forth herein.

The terminology used herein is used to describe embodiments only, and is not intended to limit the present invention. The singular expression includes the plural expression unless the context clearly dictates otherwise.

As used herein, "combination thereof" means a mixture, a laminate, a composite, a copolymer, an alloy, a blend, a reaction product, and the like of the constituents.

Herein, it should be understood that terms such as "comprises," "includes," or "have" are intended to designate the presence of an embodied feature, number, step, element, or a combination thereof, but it does not preclude the possibility of the presence or addition of one or more other features, number, step, element, or a combination thereof.

In the drawings, the thickness of layers, films, panels, regions, etc., are exaggerated for clarity and like reference numerals designate like elements throughout the specification. It will be understood that when an element such as a layer, film, region, or substrate is referred to as being "on" another element, it can be directly on the other element or intervening elements may also be present. In contrast, when an element is referred to as being "directly on" another element, there are no intervening elements present.

In addition, "layer" herein includes not only a shape formed on the whole surface when viewed from a plan view, but also a shape formed on a partial surface.

In addition, the average particle diameter and average size may be measured by a method well known to those skilled in the art, for example, may be measured by a particle size analyzer, or may be measured by a transmission electron microscopic image or a scanning electron microscopic image. Alternatively, it is possible to obtain an average particle diameter value by measuring a size using a dynamic light scattering method, performing data analysis, counting the number of particles for each particle size range, and calculating from this. Unless otherwise defined, the average particle diameter may mean the diameter (D50) of particles having a cumulative volume of 50 volume% in the particle size distribution.

"Or" is not to be construed as an exclusive meaning, for example, "A or B" is construed to include A, B, A+B, and the like.

As used herein, when specific definition is not otherwise provided, "substituted" refers to replacement of at least one hydrogen of a compound by a substituent of a halogen atom (F, Cl, Br, or I), a hydroxy group, a C1 to C20 alkoxy group, a nitro group, a cyano group, an amine group, an imino group, an azido group, an amidino group, a hydrazino group, a hydrazono group, a carbonyl group, a carbamyl group, a thiol group, an ester group, an ether group, a carboxyl group or a salt thereof, a sulfonic acid group or a salt thereof, a phosphoric acid or a salt thereof, a C1 to C20 alkyl group, a C2 to C20 alkenyl group, a C2 to C20 alkynyl group, a C6 to C30 aryl group, a C3 to C20 cycloalkyl group, a C3 to C20 cycloalkenyl group, a C3 to C20 cycloalkynyl group, a C2 to C20 heterocycloalkyl group, a C2 to C20 heterocycloalkenyl group, a C2 to C20 heterocycloalkynyl group, or a combination thereof.

As used herein, when specific definition is not otherwise provided, "heterocycloalkyl group," "heterocycloalkenyl group," "heterocycloalkynyl group" and "heterocycloalkylene group" means that at least one hetero atom of N, O, S, or P is present in the ring compound of cycloalkyl, cycloalkenyl, cycloalkynyl, and cycloalkylene, respectively.

As used herein, when a definition is not otherwise provided, in chemical formula, hydrogen is bonded at the position when a chemical bond is not drawn where supposed to be given.

As used herein, when a definition is not otherwise provided, "*" refers to a linking part between the same or different atoms, or chemical formulas.

### (Additive)

An embodiment provides an additive for a rechargeable lithium battery represented by Chemical Formula 1:

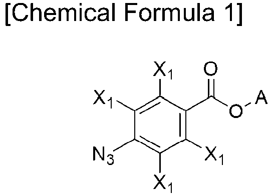

In Chemical Formula 1,
X₁ is the same or different from each other and is hydrogen or a halogen atom; and
A is a substituent represented by Chemical Formula 2, a substituent represented by Chemical Formula 3, a substituent represented by Chemical Formula 4, a substituent represented by Chemical Formula 5, or a substituted or unsubstituted C1 to C20 alkyl group; 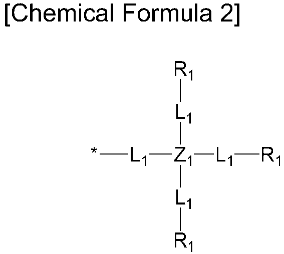
wherein, in Chemical Formula 2,
Z₁ is a carbon atom or a silicon atom,
L₁ is the same or different from each other and is a single bond or a substituted or unsubstituted C1 to C20 alkylene group; and
R₁ is the same or different from each other and is hydrogen, a substituted or unsubstituted C1 to C20 alkyl group, or a substituent represented by Chemical Formula 5; 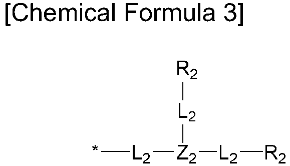
wherein, in Chemical Formula 3,
Z₂ is a nitrogen atom or a phosphorus atom;
L₂ is a single bond or a substituted or unsubstituted C1 to C20 alkylene group; and
R₂ is a hydrogen atom, a substituted or unsubstituted C1 to C20 alkyl group, or a substituent represented by Chemical Formula 5;

   [Chemical Formula 4] *-L₃-R₃
L₃ is the same or different from each other and is a single bond, a disulfide bond, a substituted or unsubstituted C1 to C20 alkylene group, or a combination thereof; and
R₃ is a hydrogen atom, a substituted or unsubstituted C1 to C20 alkyl group, or a substituent represented by Chemical Formula 5; 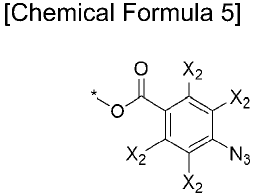
wherein, in Chemical Formula 5,
X₂ is the same or different from each other and is hydrogen or a halogen atom.

The additive of an embodiment is a compound containing at least one azide group (-N=N=N-) per molecule. The azide group decomposes at a high temperature of 140 °C or higher to generate nitrogen (N₂), which is converted to nitrene (-N).

The nitrene group reacts with other components of the rechargeable lithium battery to form a film, drastically reduces ionic conductivity, and has the effect of shutting down the rechargeable lithium battery at a temperature lower than the ignition temperature.

Accordingly, the additive of an embodiment can suppress the ignition phenomenon caused by abnormal heat generation of a rechargeable lithium battery compared to a compound that does not contain an azide group.

In addition, the additive of an embodiment corresponds to a general-purpose additive that can be used regardless of the type of rechargeable lithium battery.

Hereinafter, the additive of an embodiment will be described in detail.

Chemical Formula 1 may be represented by any one of Chemical Formulas 1-1 to 1-4: 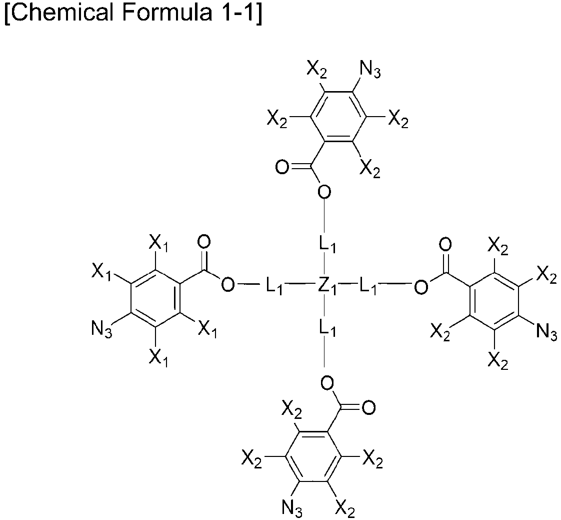 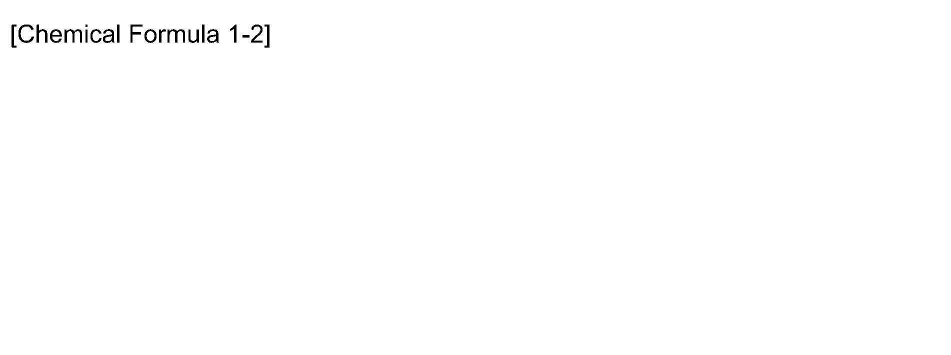 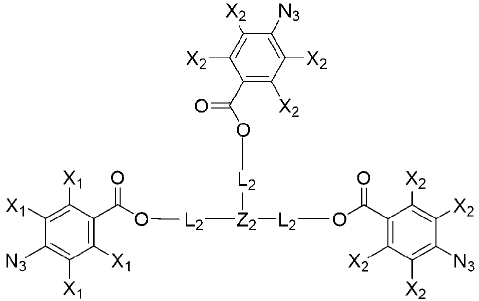 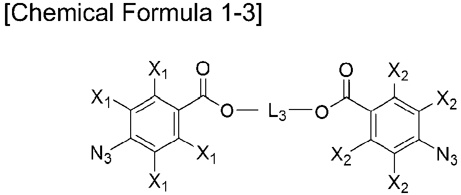 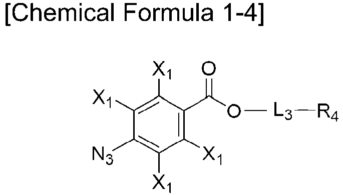
wherein, in Chemical Formulas 1-1 to 1-4,
Z₁ is a carbon atom or a silicon atom;
Z₂ is a nitrogen atom or a phosphorus atom;
X₁ is the same or different from each other and is hydrogen or a halogen atom;
X₂ is the same or different from each other and is hydrogen or a halogen atom;
L₁ is the same or different from each other and is a single bond or a substituted or unsubstituted C1 to C20 alkylene group;
L₂ is the same or different from each other and is a single bond or a substituted or unsubstituted C1 to C20 alkylene group;
L₃ is the same or different from each other and is a single bond, a disulfide bond, a substituted or unsubstituted C1 to C20 alkylene group, or a combination thereof; and
R₄ is a hydrogen atom, or a substituted or unsubstituted C1 to C20 alkyl group.
Z₁ may be a carbon atom.
Z₂ may be a nitrogen atom.
X₁s may be all hydrogen atoms or all halogen atoms.
X₂s may be all hydrogen atoms or all halogen atoms.
L₁s may be all methylene groups.
L₂s may be all ethylene groups.
L₃ may be a single bond; or may be a combination of a disulfide bond and an ethylene group.
R₄ may be a substituted or unsubstituted C1 to C20 alkyl group.

Representative examples of these additives are as follows:

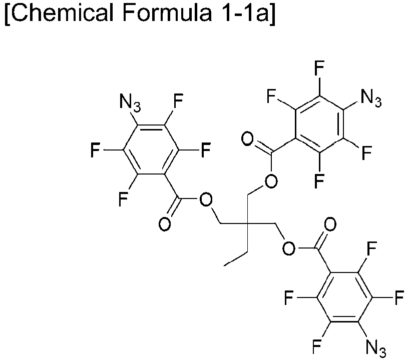

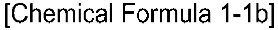

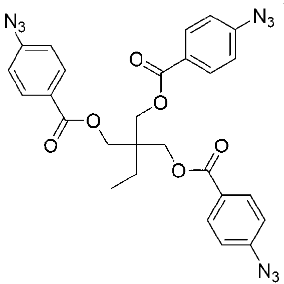

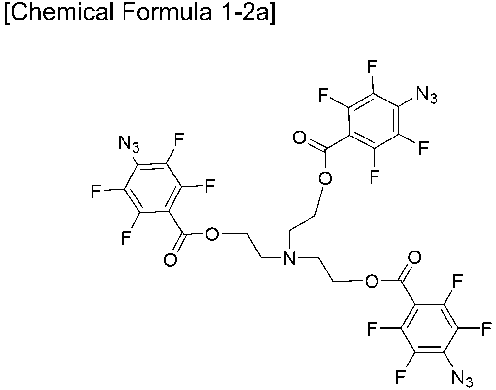

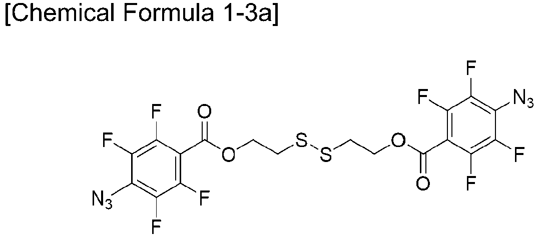

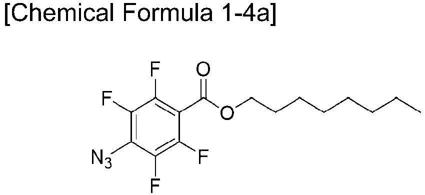

### (Electrolyte layer, Positive Electrode, Negative Electrode, and All-solid-state Battery)

In another embodiment, an electrolyte layer for a rechargeable lithium battery including the additive according to an embodiment is provided. In another embodiment, a positive electrode for a rechargeable lithium battery including the additive according to an embodiment is provided. In another embodiment, a negative electrode for a rechargeable lithium battery including the additive according to an embodiment is provided.

In another embodiment, a rechargeable lithium battery includes a positive electrode; a negative electrode; and an electrolyte layer between the positive electrode and the negative electrode, wherein at least one layer of the positive electrode, the negative electrode, and the electrolyte layer includes the additive according to an embodiment.

The rechargeable lithium battery may be an all-solid battery, a semi-solid battery, a lithium metal battery, or a lithium ion battery.

### All-solid-state Battery

As an example, the rechargeable lithium battery may be an all-solid-state battery. The all-solid-state battery may also be expressed as an all-solid-state rechargeable battery or an all-solid rechargeable lithium battery.

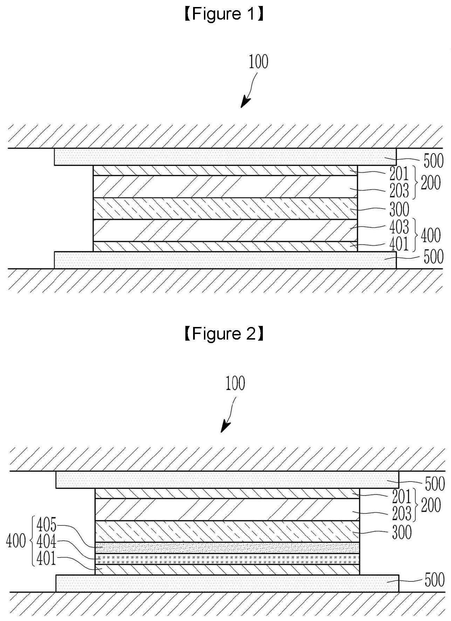

FIG. 1 is a cross-sectional view of an all-solid-state battery according to an embodiment. Referring to FIG. 1, the all-solid-state battery 100 may have a structure that an electrode assembly, in which a negative electrode 400 including a negative current collector 401 and a negative electrode active material layer 403, a solid electrolyte layer 300, and a positive electrode 200 including a positive electrode active material layer 203 and a positive electrode current collector 201 are stacked, is inserted into a case such as a pouch and the like. The all-solid-state battery 100 may further include at least one elastic layer 500 on the outside of at least either one of the positive electrode 200 and the negative electrode 400. FIG. 1 shows one electrode assembly including the negative electrode 400, the solid electrolyte layer 300, and the positive electrode 200, but two or more electrode assemblies may be stacked to manufacture an all-solid-state battery.

An all-solid-state battery according to an embodiment can be manufactured by preparing a stack by sequentially stacking a positive electrode, a solid electrolyte, and a negative electrode, and pressurizing the stack.

The pressurizing may be performed at a temperature of, for example, 25 °C to 90 °C, and may be performed at a pressure of less than or equal to 550 MPa, or less than or equal to 500 MPa, for example, 400 MPa to 500 MPa. The pressurizing may be, for example, isostatic press, roll press, or plate press.

The all-solid-state battery may be a unit cell having a structure of positive electrode/solidelectrolyte layer/negative electrode, a bicell having a structure of positive electrode/solid electrolyte layer/negative electrode/solid electrolyte layer/positive electrode, or a stacked battery in which the structures of the unit cell are repeated.

The shape of the all-solid-state battery is not particularly limited, and may be, for example, coin-shaped, button-shaped, sheet-shaped, stacked-shaped, cylindrical, flat, etc. Additionally, the all-solid-state battery may also be applied to medium to large-sized batteries used in electric vehicles, etc. For example, the all-solid-state battery may also be used in hybrid vehicles such as plug-in hybrid electric vehicles (PHEV). In addition, it may be applied to an energy storage system (ESS) that requires large amounts of power storage, and may also be applied to electric bicycles or power tools.

### Solid Electrolyte Layer

The solid electrolyte layer includes a solid electrolyte. In this case, the electrolyte layer may be a solid electrolyte layer.

The electrolyte layer may include the additive. Based on a total amount of 100 wt% of the electrolyte layer, the additive may be included in an amount of 0.1 to 10 wt%, or 0.1 to 5 wt%. Additionally, a weight ratio of solid electrolyte : additive in the electrolyte layer may be 99.9:0.01 to 90:10, or 99:1 to 90:10. In this range, a shutdown effect by the additive may be properly implemented.

The solid electrolyte may be a type of inorganic solid electrolyte, and may include, for example, a sulfide-based solid electrolyte, an oxide-based solid electrolyte, a halide-based solid electrolyte, or a combination thereof.

### Sulfide-based Solid Electrolyte

The sulfide-based solid electrolyte may include, for example Li₂S-P₂S₅, Li₂S-P₂S₅-LiX (wherein X is a halogen element, for example I or Cl), Li₂S-P₂S₅-Li₂O, Li₂S-P₂S₅-Li₂O-LiI, Li₂S-SiS₂, Li₂S-SiS₂-LiI, Li₂S-SiS₂-LiBr, Li₂S-SiS₂-LiCl, Li₂S-SiS₂-B₂S₃-LiI, Li₂S-SiS₂-P₂S₅-LiI, Li₂S-B₂S₃, Li₂S-P₂S₅-ZₘSₙ (wherein m and n are an integer, respectively, and Z is Ge, Zn, or Ga), Li₂S-GeS₂, Li₂S-SiS₂-Li₃PO₄, Li₂S-SiS₂-LiₚMO_{q} (wherein p and q are integers, and M is P, Si, Ge, B, Al, Ga, or In), or a combination thereof.

The sulfide-based solid electrolyte may be obtained by, for example, mixing Li₂S and P₂S₅ in a molar ratio of 50:50 to 90:10 or 50:50 to 80:20 and optionally performing heat-treatment. Within the above mixing ratio range, a sulfide-based solid electrolyte having excellent ionic conductivity may be prepared. The ionic conductivity may be further improved by adding SiS₂, GeS₂, B₂S₃, and the like as other components thereto.

Mechanical milling or a solution method may be applied as a mixing method of sulfur-containing raw materials for preparing a sulfide-based solid electrolyte. The mechanical milling is to make starting materials into particulates by putting the starting materials in a ball mill reactor and fervently stirring them. The solution method may be performed by mixing the starting materials in a solvent to obtain a solid electrolyte as a precipitate. In addition, in the case of heat-treatment after mixing, crystals of the solid electrolyte may be more robust and ionic conductivity may be improved. For example, the sulfide-based solid electrolyte may be prepared by mixing sulfur-containing raw materials and performing heat treatment two or more times. In this case, a sulfide-based solid electrolyte having high ionic conductivity and robustness may be prepared.

The sulfide-based solid electrolyte particles according to an embodiment, for example, may be prepared through a first heat treatment of mixing sulfur-containing raw materials and firing at 120 °C to 350 °C and a second heat treatment of mixing the resultant of the first heat treatment and firing the same at 350 °C to 800 °C. The first heat treatment and the second heat treatment may be performed in an inert gas or nitrogen atmosphere, respectively. The first heat treatment may be performed for 1 hour to 10 hours, and the second heat treatment may be performed for 5 hours to 20 hours. Small raw materials may be milled through the first heat treatment, and a final solid electrolyte can be synthesized through the second heat treatment. Through such two or more heat treatments, a sulfide-based solid electrolyte having high ionic conductivity and high performance can be obtained, and such a solid electrolyte may be suitable for mass production. The temperature of the first heat treatment may be, for example, 150 °C to 330 °C, or 200 °C to 300 °C, and the temperature of the second heat treatment may be, for example, 380 °C to 700 °C, or 400 °C to 600 °C

For example, the sulfide-based solid electrolyte particles may include argyrodite-type sulfide. The argyrodite-type sulfide-based solid electrolyte particle may have high ionic conductivity close to the range of 10⁻⁴ to 10⁻² S/cm, which is the ionic conductivity of general liquid electrolytes at room temperature, and may form an intimate bond between the positive electrode active material and the solid electrolyte without causing a decrease in ionic conductivity, and furthermore, an intimate interface between the electrode layer and the solid electrolyte layer. An all-solid-state rechargeable battery including the same may have improved battery performance such as rate capability, coulombic efficiency, and cycle-life characteristics.

For example, the argyrodite-type sulfide-based solid electrolyte particles may include a compound represented by Chemical Formula 21.

[Chemical Formula 21] (LiₐM¹_{b}M²_{c})(P_{d}M³ₑ)(S_{f}M⁴_{g})Xₕ

In Chemical Formula 21, 4≤a≤8, M¹ is Mg, Cu, Ag, or a combination thereof, 0≤b<0.5, M² is Na, K, or a combination thereof, 0≤c<0.5, M³ is Sn, Zn, Si, Sb, Ge, or a combination thereof, 0<d<4, 0≤e<1, M⁴ is O, SOₙ, or a combination thereof, 1.5≤n≤5, 3≤f≤12, 0≤g<2, X is F, Cl, Br, I, or a combination thereof, and 0≤h≤2.

For example, in Chemical Formula 21, a halide element (X) may be necessarily included, and in this case, it may be expressed as 0<h≤2. For example, M¹ element may be necessarily included in Chemical Formula 21, and in this case, it may be expressed as 0<b<0.5. In Chemical Formula 21, M³ may be understood as an element substituted for P and may be 0<e<1. In Chemical Formula 21, M⁴ is substituted for S and, for example, may be 0<g<2, and f, a ratio of S, may be, for example, 3≤f≤7. When M⁴ is SOₙ, SOₙ may be, for example S₄O₆, S₃O₆, S₂O₃, S₂O₄, S₂O₅, S₂O₆, S₂O₇, S₂O₈, SO₄, or SO₅, and for example, may be SO₄.

For example, in Chemical Formula 21, a+b+c+h=7, d+e=1, and f+g+h=6.

As a specific example, the argyrodite-type sulfide-based solid electrolyte particles may include Li₃PS₄, Li₇P₃S₁₁, Li₇PS₆, Li₆PS₅Cl, Li₆PS₅Br, Li_{5.8}PS_{4.8}Cl_{1.2}, Li_{6.2}PS_{5.2}Br_{0.8}, Li_{5.75}PS_{4.75}Cl_{1.25}, (Li_{5.69}Cu_{0.06})PS_{4.75}Cl_{1.25}, (Li_{5.72}Cu_{0.03})PS_{4.75}Cl_{1.25}, (Li_{5.69}Cu_{0.06})P(S_{4.70}(SO₄)_{0.05})Cl_{1.25}, (Li_{5.69}Cu_{0.06})P(S_{4.60}(SO₄)_{0.15})Cl_{1.25}, (Li_{5.72}Cu_{0.03})P(S_{4.725}(SO₄)_{0.025})Cl_{1.25}, (Li_{5.72}Na_{0.03})P(S_{4.725}(SO₄)_{0.025})Cl_{1.25}, Li_{5.75}P(S_{4.725}(SO₄)_{0.025})Cl_{1.25}, or a combination thereof, but are not limited thereto.

The argyrodite-type sulfide-based solid electrolyte may be prepared, for example, by mixing lithium sulfide and phosphorus sulfide, and optionally lithium halide. Heat treatment may be performed after mixing them. The heat treatment may include, for example, two or more heat treatment steps. The method of preparing the argyrodite-type sulfide-based solid electrolyte may include, for example, a first heat treatment in which raw materials are mixed and fired at 120 °C to 350 °C, and a second heat treatment in which the resultant of the first heat treatment is mixed again and fired at 350 °C to 800 °C.

An average particle diameter (D50) of the sulfide-based solid electrolyte particles may be, for example, 0.1 µm to 5.0 µm or 0.1 µm to 3.0 µm, and may be small particles of 0.1 µm to 1.9 µm or large particles of 2.0 µm to 5.0 µm. The sulfide-based solid electrolyte particles may be a mixture of small particles with an average particle diameter of 0.1 µm to 1.9 µm and large particles with an average particle diameter of 2.0 µm to 5.0 µm. The average particle diameter of the sulfide-based solid electrolyte particles may be measured using an electron microscopic image, and for example, a particle size distribution may be obtained by measuring the size (diameter or long axis length) of about 20 particles in a scanning electron microscopic image, and D50 may be calculated therefrom.

### Oxide-based Solid Electrolyte

The oxide-based solid electrolyte may include, for example, Li₁₊ₓTi₂₋ₓAl(PO₄)₃ (LTAP) (0≤x≤4), Li_{1+x+y}AlₓTi₂₋ₓSi_{y}P_{3-y}O₁₂ (0<x<2, 0≤y<3), BaTiO₃, Pb(Zr,Ti)O₃ (PZT), Pb₁₋ₓLaₓZr_{1-y}Ti_{y}O₃ (PLZT) (0≤x<1, 0≤y<1), PB(Mg₃Nb_{2/3})O₃-PbTiO₃ (PMN-PT), HfO₂, SrTiO₃, SnO₂, CeO₂, Na₂O, MgO, NiO, CaO, BaO, ZnO, ZrO₂, Y₂O₃, Al₂O₃, TiO₂, SiO₂, lithium phosphate (Li₃PO₄), lithium titanium phosphate (LiₓTi_{y}(PO₄)₃, 0<x<2, 0<y<3), Li_{1+x+y}(Al, Ga)ₓ(Ti, Ge)₂₋ₓSi_{y}P_{3-y}O₁₂ (0≤x≤1, 0≤y≤1), lithium lanthanum titanate (LiₓLa_{y}TiO₃, 0<x<2, 0<y<3), Li₂O, LiAlO₂, Li₂O-Al₂O₃-SiO₂-P₂O₅-TiO₂-GeO₂-based ceramics, Garnet-based ceramics Li₃₊ₓLa₃M₂O₁₂ (wherein M = Te, Nb, or Zr; x is an integer of 1 to 10), or a mixture thereof.

### Halide-based Solid Electrolyte

The solid electrolyte layer may further include, for example, a halide-based solid electrolyte. The halide-based solid electrolyte may include a halogen element as a main component, meaning that a ratio of the halide element to all elements constituting the solid electrolyte is 50 mol% or more, 70 mol% or more, 90 mol% or more, or 100 mol%. As an example, the halide-based solid electrolyte may not include sulfur element.

The halide-based solid electrolyte may include a lithium element, a metal element other than lithium, and a halogen element. The metal element other than lithium may include Al, As, B, Bi, Ca, Cd, Co, Cr, Fe, Ga, Hf, In, Mg, Mn, Ni, Sb, Sc, Sn, Ta, Ti, Y, Zn, Zr, or a combination thereof. The halogen element may be F, Cl, Br, I, or a combination thereof, and for example, it may be Cl, Br, or a combination thereof. The halide-based solid electrolyte may be, for example, represented by LiₐM₁X₆ (M is Al, As, B, Bi, Ca, Cd, Co, Cr, Fe, Ga, Hf, In, Mg, Mn, Ni, Sb, Sc, Sn, Ta, Ti, Y, Zn, Zr, or a combination thereof, X is F, Cl, Br, I, or a combination thereof, and 2≤a≤3). The halide-based solid electrolyte may include, for example, Li₂ZrCl₆, Li_{2.7}Y_{0.7}Zr_{0.3}Cl₆, Li_{2.5}Y_{0.5}Zr_{0.5}Cl₆, Li_{2.5}In_{0.5}Zr_{0.5}Cl₆, Li₂In_{0.5}Zr_{0.5}Cl₆, Li₃YBr₆, Li₃YCl₆, Li₃YBr₂Cl₄, Li₃YbCl₆, Li_{2.6}Hf_{0.4}Yb_{0.6}Cl₆, or a combination thereof, but is not limited thereto.

### Binder

The solid electrolyte layer may further include a binder. The binder may include, for example, a nitrile-butadiene rubber, a hydrogenated nitrile-butadiene rubber, a styrene-butadiene rubber, an acrylated styrene-butadiene rubber, an acrylonitrile-butadiene rubber, an acrylic rubber, a butyl rubber, a fluorine rubber, a natural rubber, polydimethylsiloxane, polyethyleneoxide, polyvinylpyrrolidone, polyvinylpyridine, chlorosulfonated polyethylene, polyvinyl alcohol, polytetrafluoroethylene, polyvinylidene fluoride, a polyvinylidene fluoride-hexafluoropropylene copolymer, polyvinylchloride, carboxylated polyvinylchloride, polyvinylfluoride, polyethylene, polypropylene, an ethylene-propylene copolymer, an ethylene-propylene-diene copolymer, polyamideimide, polyimide, poly(meth)acrylate, polyacrylonitrile, polystyrene, polyurethane, a copolymer thereof, or a combination thereof.

The binder may be included in an amount of 0.1 wt% to 3 wt%, for example, 0.5 wt% to 2 wt%, or 0.5 wt% to 1.5 wt%, based on 100 wt% of the solid electrolyte layer. When the binder is included in the above range, the components in the solid electrolyte layer may be well combined without reducing the ionic conductivity of the solid electrolyte, thereby improving durability and reliability of the battery.

### Other Components

The solid electrolyte layer may further optionally include an alkali metal salt, and/or an ionic liquid, and/or a conductive polymer.

The alkali metal salt may be, for example, a lithium salt. A content of the lithium salt in the solid electrolyte layer may be greater than or equal to 1 M, for example, 1 M to 4 M. In this case, the lithium salt may improve ionic conductivity by improving lithium ion mobility of the solid electrolyte layer.

The lithium salt may be applied without type limitations, and may include, for example, LiPF₆, LiBF₄, LiSbF₆, LiAsF₆, LiClO₄, LiAlO₂, LiAlCl₄, LiPO₂F₂, LiCl, LiI, LiSCN, LiN(CN)₂, lithium bis(oxalato)borate (LiBOB), lithium difluoro (oxalato)borate (LiDFOB), lithium difluorobis(oxalato)phosphate (LiDFBP), lithium bis(trifluoromethanesulfonyl)imide (LiTFSI), lithium bis(fluoro)sulfonyl)imide (LiFSI), lithium bis(pentafluoroethanesulfonyl)imide (LiBETI), lithium trifluoromethane sulfonate, lithium tetrafluoroethane sulfonate, or a combination thereof.

For example, the lithium salt may be an imide-based lithium salt such as LiTFSI, LiFSI, LiBETI, or a combination thereof. The imide-based lithium salt may maintain or improve ionic conductivity by appropriately maintaining chemical reactivity with the ionic liquid.

The ionic liquid has a melting point below room temperature, so it is in a liquid state at room temperature and refers to a salt or room temperature molten salt composed of ions alone.

The ionic liquid may be a compound including at least one cation selected from a) ammonium-based, pyrrolidinium-based, pyridinium-based, pyrimidinium-based, imidazolium-based, piperidinium-based, pyrazolium-based, oxazolium-based, pyridazinium-based, phosphonium-based, sulfonium-based, triazolium-based, and a mixture thereof, and b) at least one anion selected from BF₄⁻, PF₆⁻, AsF₆⁻, SbF₆⁻, AlCl₄⁻, HSO₄⁻, ClO₄⁻, CH₃SO₃⁻, CF₃CO₂⁻, Cl⁻, Br⁻, I⁻, BF₄⁻, SO₄⁻, CF₃SO₃⁻, (FSO₂)₂N⁻, (C₂F₅SO₂)₂N⁻, (C₂F₅SO₂)(CF₃SO₂)N⁻, and (CF₃SO₂)₂N⁻.

The ionic liquid may be, for example, one or more selected from N-methyl-N-propylpyrrolidinium bis(trifluoromethanesulfonyl)imide, N-butyl-N-methylpyrrolidium bis(3-trifluoromethylsulfonyl) imide, 1-butyl-3-methylimidazolium bis(trifluoromethylsulfonyl)amide, and 1-ethyl-3-methylimidazolium bis(trifluoromethylsulfonyl)amide.

A weight ratio of the solid electrolyte and the ionic liquid in the solid electrolyte layer may be 0.1:99.9 to 90:10, for example, 10:90 to 90:10, 20:80 to 90:10, 30:70 to 90:10, 40:60 to 90:10, or 50:50 to 90:10. The solid electrolyte layer satisfying the above ranges may maintain or improve ionic conductivity by improving the electrochemical contact area with the electrode. Accordingly, the energy density, discharge capacity, rate capability, etc. of the all-solid-state rechargeable battery may be improved.

### Positive Electrode

In an embodiment, it includes a current collector and a positive electrode active material layer on the current collector, and the positive electrode active material layer includes a positive electrode active material and may optionally include a solid electrolyte, a binder, and/or a conductive material. Additionally, the positive electrode active material layer may include the additive.

### Positive Electrode Active Material

The positive electrode active material may be a compound (lithiated intercalation compound) capable of intercalating and deintercalating lithium. Specifically, one or more types of composite oxides of lithium and a metal selected from cobalt, manganese, nickel, and combinations thereof may be used.

The composite oxide may be a lithium transition metal composite oxide, and specific examples may include lithium nickel-based oxide, lithium cobalt-based oxide, lithium manganese-based oxide, a lithium iron phosphate-based compound, cobalt-free nickel-manganese-based oxide, overlithiated layered oxide, or a combination thereof.

As an example, the positive electrode active material may be a high nickel-based positive electrode active material having a nickel content of greater than or equal to 80 mol% based on 100 mol% of metals excluding lithium in the lithium transition metal composite oxide. The nickel content in the high nickel-based positive electrode active material may be greater than or equal to 85 mol%, greater than or equal to 90 mol%, greater than or equal to 91 mol%, or greater than or equal to 94 mol%, and less than or equal to 99 mol% based on 100 mol% of metals excluding lithium. The high-nickel-based positive electrode active materials may achieve high capacity and may be applied to high-capacity, high-density rechargeable lithium batteries.

As a more specific example, a compound represented by any of the following chemical formulas may be used. LiₐA_{1-b}X_{b}O_{2-c}D_{c} (0.90 ≤ a ≤ 1.8, 0 ≤ b ≤ 0.5, 0 ≤ c ≤ 0.05); LiₐMn_{2-b}X_{b}O_{4-c}D_{c} (0.90 ≤ a ≤ 1.8, 0 ≤ b ≤ 0.5, 0 ≤ c ≤ 0.05); LiₐNi_{1-b-c}Co_{b}X_{c}O_{2-α}D_{α} (0.90 ≤ a ≤ 1.8, 0 ≤ b ≤ 0.5, 0 ≤ c ≤0.5, 0 < α < 2);LiₐNi_{1-b-c}Mn_{b}X_{c}O_{2-α}D_{α} (0.90 ≤ a ≤ 1.8, 0 ≤ b ≤ 0.5, 0 ≤ c ≤ 0.5, 0 < α < 2);LiₐNi_{b}Co_{c}L¹_{d}GₑO₂ (0.90 ≤ a ≤ 1.8, 0 ≤ b ≤ 0.9, 0 ≤ c ≤ 0.5, 0 ≤ d ≤ 0.5, 0 ≤ e ≤ 0.1); LiₐNiG_{b}O₂ (0.90 ≤ a ≤ 1.8, 0.001 ≤ b ≤ 0.1); LiₐCoG_{b}O₂ (0.90 ≤ a ≤ 1.8, 0.001 ≤ b ≤ 0.1); LiₐMn_{1-b}G_{b}O₂ (0.90 ≤ a ≤ 1.8, 0.001 ≤ b ≤ 0.1); LiₐMn₂G_{b}O₄ (0.90 ≤ a ≤1.8, 0.001 ≤ b ≤ 0.1); LiₐMn_{1-g}G_{g}PO₄ (0.90 ≤ a ≤ 1.8, 0 ≤ g ≤ 0.5); Li_{(3-f)}Fe₂(PO₄)₃ (0 ≤ f ≤ 2); LiₐFePO₄ (0.90 ≤ a ≤ 1.8)

In the above chemical formulas, A is Ni, Co, Mn, or a combination thereof; X is Al, Ni, Co, Mn, Cr, Fe, Mg, Sr, V, a rare earth element, or a combination thereof; D is O, F, S, P, or a combination thereof; G is Al, Cr, Mn, Fe, Mg, La, Ce, Sr, V, or a combination thereof; Q is Ti, Mo, Mn, or a combination thereof; Z is Cr, V, Fe, Sc, Y, or a combination thereof; and L¹ is Mn, Al, or a combination thereof.

The positive electrode active material may be, for example, lithium nickel-based oxide represented by Chemical Formula 11, lithium cobalt-based oxide represented by Chemical Formula 12, a lithium iron phosphate-based compound represented by Chemical Formula 13, and cobalt-free lithium nickel manganese-based oxide represented by Chemical Formula 14, or a combination thereof.

[Chemical Formula 11] Liₐ₁Niₓ₁M¹_{y1}M²_{z1}O_{2-b1}X_{b1}

In Chemical Formula 11, 0.9≤a1≤1.8, 0.3≤x1≤1, 0≤y1≤0.7, 0≤z1≤0.7, 0.9≤x1+y1+z1≤1.1, and 0≤b1≤0.1, M¹ and M² are each independently one or more elements selected from Al, B, Ba, Ca, Ce, Co, Cr, Cu, Fe, Mg, Mn, Mo, Nb, Si, Sn, Sr, Ti, V, W, and Zr, and X is one or more elements selected from F, P, and S.

In Chemical Formula 1, 0.6≤x1≤1, 0≤y1≤0.4, and 0≤z1≤0.4, or 0.8≤x1≤1, 0≤y1≤0.2, and 0≤z1≤0.2.

[Chemical Formula 12] Liₐ₂Coₓ₂M³_{y2}O_{2-b2}X_{b2}

In Chemical Formula 12, 0.9≤a2≤1.8, 0.7≤x2≤1, 0≤y2≤0.3, 0.9≤x2+y2≤1.1, and 0≤b2≤0.1, M³ is one or more elements selected from Al, B, Ba, Ca, Ce, Cr, Cu, Fe, Mg, Mn, Mo, Ni, Se, Si, Sn, Sr, Ti, V, W, Y, Zn, and Zr, and X is one or more elements selected from F, P, and S.

[Chemical Formula 13] Liₐ₃Feₓ₃M⁴_{y3}PO_{4-b3}X_{b3}

In Chemical Formula 13, 0.9≤a3≤1.8, 0.6≤x3≤1, 0≤y3≤0.4, and 0≤b3≤0.1, M⁴ is one or more elements selected from Al, B, Ba, Ca, Ce, Co, Cr, Cu, Mg, Mn, Mo, Ni, Se, Si, Sn, Sr, Ti, V, W, Y, Zn, and Zr, and X is one or more elements selected from F, P, and S.

[Chemical Formula 14] Liₐ₄Niₓ₄Mn_{y4}M⁵_{z4}O_{2-b4}X_{b4}

In Chemical Formula 14, 0.9≤a2≤1.8, 0.8≤x4<1, 0<y4≤0.2, 0≤z4≤0.2, 0.9≤x4+y4+z4≤1.1, and 0≤b4≤0.1, M⁵ is one or more elements selected from Al, B, Ba, Ca, Ce, Cr, Fe, Mg, Mo, Nb, Si, Sn, Sr, Ti, V, W, and Zr, and X is one or more elements selected from F, P, and S.

An average particle diameter (D50) of the positive electrode active material may be 1 µm to 25 µm, for example 3 µm to 25 µm, 1 µm to 20 µm, 1 µm to 18 µm, 3 µm to 15 µm, or 5 µm to 15 µm. As an example, the positive electrode active material may include small particles having an average particle diameter (D50) of 1 µm to 9 µm and large particles having an average particle diameter (D50) of 10 µm to 25 µm. A positive electrode active material having this particle size range can be harmoniously mixed with other components within the positive electrode active material layer and can achieve high capacity and high energy density. Herein, the average particle diameter may be obtained by selecting about 20 particles at random among particles in a scanning electron microscopic image of the positive electrode active material, measuring the particle diameter (diameter, long axis, or length of the long axis) to obtain the particle size distribution, and taking the diameter (D50) of particles with a cumulative volume of 50 volume% as the average particle diameter in the particle size distribution.

The positive electrode active material may be in the form of secondary particles made by agglomerating a plurality of primary particles, or may be in the form of single particles. Additionally, the positive electrode active material may have a spherical or close to spherical shape, or may have a polyhedral or irregular shape.

Meanwhile, the positive electrode active material may include a buffer layer on the particle surface. The buffer layer can be expressed as a coating layer, a protective layer, etc., and can play a role in lowering the interfacial resistance between the positive electrode active material and the sulfide-based solid electrolyte particles. As an example, the buffer layer may include lithium-metal-oxide, where the metal may be, for example, one or more elements selected from Al, B, Ca, Ce, Cr, Fe, Mg, Mo, Nb, Si, Sn, Sr, Ta, V, W, and Zr. The lithium-metal-oxide may improve the performance of the positive electrode active material by facilitating the movement of lithium ions and electronic conduction, while lowering the interfacial resistance between the positive electrode active material and solid electrolyte particles.

The positive electrode active material may be included in an amount of 55 wt% to 99.5 wt%, for example 65 wt% to 95 wt%, or 75 wt% to 91 wt%, based on 100 wt% of the positive electrode active material layer.

### Binder

The binder serves to attach the positive electrode active material particles well to each other and also to attach the positive electrode active material well to the current collector. Examples of the binder may include polyvinyl alcohol, carboxymethyl cellulose, hydroxypropyl cellulose, diacetyl cellulose, polyvinylchloride, carboxylated polyvinylchloride, polyvinylfluoride, a polymer including ethylene oxide, polyvinylpyrrolidone, polyurethane, polytetrafluoroethylene, polyvinylidene fluoride, polyethylene, polypropylene, a styrene-butadiene rubber, a (meth)acrylated styrene-butadiene rubber, an epoxy resin, a (meth)acrylic resin, a polyester resin, nylon, and the like, as non-limiting examples.

### Conductive Material

The conductive material may be used to impart conductivity to the electrode. Any material that does not cause chemical change and conducts electrons can be used in the battery. Examples of the conductive material may include a carbon-based material such as natural graphite, artificial graphite, carbon black, acetylene black, ketjen black, a carbon fiber, a carbon nanofiber, and carbon nanotube; a metal-based material containing copper, nickel, aluminum, silver, etc., in a form of a metal powder or a metal fiber; a conductive polymer such as a polyphenylene derivative; or a mixture thereof.

A content of the binder and the conductive material may be 0.5 wt% to 5 wt%, respectively, based on 100 wt% of the positive electrode active material layer.

The positive electrode active material layer may optionally further include a solid electrolyte. The solid electrolyte may include, for example, a sulfide-based solid electrolyte, an oxide-based solid electrolyte, a halide-based solid electrolyte, or a combination thereof, and detailed descriptions thereof will be provided later in the solid electrolyte layer section.

Based on 100 wt% of the positive electrode active material layer, the solid electrolyte may be included in an amount of 0.1 wt% to 35 wt%, for example, 1 wt% to 35 wt%, 5 wt% to 30 wt%, and 8 wt% to 25 wt%, or 10 wt% to 20 wt%.

In the positive electrode active material layer, based on a total of 100 wt% of the positive electrode active material and solid electrolyte, 65 wt% to 99 wt% of the positive electrode active material and 1 wt% to 35 wt% of the solid electrolyte may be included, and for example, 80 wt% to 90 wt% of the positive electrode active material and 10 wt% to 20 wt% of solid electrolyte may be included. When the solid electrolyte is included in the positive electrode at this amount, the efficiency and cycle-life characteristics of the all-solid-state rechargeable battery can be improved without reducing the capacity.

The current collector may include Al, but is not limited thereto.

### Negative Electrode

For example, a negative electrode for an all-solid-state battery may include a current collector and a negative electrode active material layer on the current collector. The negative electrode active material layer includes a negative electrode active material and may further include a binder, a conductive material, and/or a solid electrolyte. Additionally, the negative electrode active material layer may include the additive.

The negative electrode active material may be a material that reversibly intercalates/deintercalates lithium ions, a lithium metal, a lithium metal alloy, a material capable of doping and dedoping lithium, or a transition metal oxide.

The material that reversibly intercalates/deintercalates lithium ions may include a carbon-based negative electrode active material, for example, crystalline carbon, amorphous carbon or a combination thereof. The crystalline carbon may be graphite such as non-shaped, sheet-shaped, flake-shaped, sphere-shaped, or fiber-shaped natural graphite or artificial graphite. The amorphous carbon may be a soft carbon, a hard carbon, a mesophase pitch carbonization product, calcined coke, and the like.

The lithium metal alloy may include lithium and one or more metals selected from Na, K, Rb, Cs, Fr, Be, Mg, Ca, Sr, Si, Sb, Pb, In, Zn, Ba, Ra, Ge, Al, and Sn.

The material capable of doping/dedoping lithium may be a Si-based negative electrode active material or a Sn-based negative electrode active material, the Si-based negative electrode active material may include silicon, a silicon-carbon composite, SiOₓ (0<x<2), a Si-Q alloy (wherein Q is an element selected from an alkali metal, an alkaline-earth metal, a Group 13 element, a Group 14 element, a Group 15 element, a Group 16 element, a transition metal, a rare earth element, and a combination thereof, but not Si), and the Sn-based negative electrode active material may include Sn, SnO₂, a Sn-R alloy (wherein R is an element selected from an alkali metal, an alkaline-earth metal, a Group 13 element, a Group 14 element, a Group 15 element, a Group 16 element, a transition metal, a rare earth element, and a combination thereof, but not Sn), and at least one of these may be mixed with SiO₂. The elements Q and R may be selected from Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Tl, Ge, P, As, Sb, Bi, S, Se, Te, Po, and a combination thereof.

For example, the silicon-carbon composite may be a silicon-carbon composite including a core including crystalline carbon and silicon particles and an amorphous carbon coating layer on the surface of the core. The crystalline carbon may be artificial graphite, natural graphite, or a combination thereof. The amorphous carbon precursor may be coal-based pitch, mesophase pitch, petroleum-based pitch, coal-based oil, petroleum-based heavy oil, or a polymer resin such as phenol resin, furan resin, and polyimide resin. Herein, a content of silicon may be 10 wt% to 50 wt% based on a total weight of the silicon-carbon composite. In addition, a content of the crystalline carbon may be 10 wt% to 70 wt% based on a total weight of the silicon-carbon composite, and a content of the amorphous carbon may be 20 wt% to 40 wt% based on a total weight of the silicon-carbon composite. Additionally, a thickness of the amorphous carbon coating layer may be 5 nm to 100 nm.

An average particle diameter (D50) of the silicon particles may be 10 nm to 20 µm, for example, 10 nm to 200 nm. The silicon particles may exist in an oxidized form, and in this case, the atomic content ratio of Si:O in the silicon particles, which indicates a degree of oxidation, may be 99:1 to 33:67. The silicon particles may be SiOₓ particles, and in this case, the x range in SiOx may be greater than 0 and less than 2.

The Si-based negative electrode active material or the Sn-based negative electrode active material may be used in combination with a carbon-based negative electrode active material. A mixing ratio of the Si-based negative electrode active material or Sn-based negative electrode active material; and a carbon-based negative electrode active material; may be 1:99 to 90:10.

In the negative electrode active material layer, the negative electrode active material may be included in an amount of 95 wt% to 99 wt% based on a total weight of the negative electrode active material layer.

In an embodiment, the negative electrode active material layer further includes a binder, and may optionally further include a conductive material. The content of the binder in the negative electrode active material layer may be 1 wt% to 5 wt%% based on a total weight of the negative electrode active material layer. In addition, when the conductive material is further included, the negative electrode active material layer may include 90 wt% to 98 wt% of the negative electrode active material, 1 wt% to 5 wt% of the binder, and 1 wt% to 5 wt% of the conductive material.

The binder may serve to attach the negative electrode active material particles well to each other and also to attach the negative electrode active material well to the current collector. The binder may be a water-insoluble binder, a water-soluble binder, or a combination thereof.

The water-insoluble binder may be, for example polyvinyl chloride, carboxylated polyvinyl chloride, polyvinyl fluoride, an ethylene oxide-containing polymer, an ethylene propylene copolymer, polystyrene, polyvinylpyrrolidone, polyurethane, polytetrafluoro ethylene, polyvinylidene fluoride, polyethylene, polypropylene, polyamideimide, polyimide, or a combination thereof.

The water-soluble binder may include a rubber binder or a polymer resin binder. The rubber binder may be selected from a styrene-butadiene rubber, an acrylated styrene-butadiene rubber, an acrylonitrile-butadiene rubber, an acrylic rubber, a butyl rubber, a fluororubber, and a combination thereof. The polymer resin binder may be selected from polyethylene oxide, polyvinylpyrrolidone, polyepichlorohydrin, polyphosphazene, polyacrylonitrile, an ethylene propylene diene copolymer, polyvinylpyridine, chlorosulfonated polyethylene, latex, a polyester resin, an acrylic resin, a phenol resin, an epoxy resin, polyvinyl alcohol, and a combination thereof.

When a water-soluble binder is used as the negative electrode binder, a cellulose-based compound viscosity capable of imparting viscosity may be used. The cellulose-based compound may include carboxymethyl cellulose, hydroxypropylmethyl cellulose, methyl cellulose, an alkali metal salt thereof, or a mixture thereof. The alkali metal may be Na, K, or Li. An amount of such a thickener used may be 0.1 parts by weight to 3 parts by weight based on 100 parts by weight of the negative electrode active material.

The conductive material is used to provide conductivity to the electrode, and any material that has electronic conductivity without causing chemical change can be used. The conductive material may include, for example a carbon-based material such as natural graphite, artificial graphite, carbon black, acetylene black, ketjen black, a carbon fiber, a carbon nanofiber, carbon nanotube, and the like; a metal-based material of a metal powder or a metal fiber including copper, nickel, aluminum silver, and the like; a conductive polymer such as a polyphenylene derivative; or a mixture thereof.

The negative electrode current collector may include, for example, a copper foil, a nickel foil, a stainless steel foil, a titanium foil, a nickel foam, a copper foam, a polymer substrate coated with a conductive metal, or a combination thereof.

Meanwhile, as an example, the negative electrode for the all-solid-state battery may be a precipitation-type negative electrode. The precipitation-type negative electrode may be a negative electrode which has no negative electrode active material during the assembly of a battery but in which a lithium metal and the like are precipitated during the charge of the battery and serve as a negative electrode active material.

FIG. 2 is a schematic cross-sectional view of an all-solid-state battery including a precipitation-type negative electrode. Referring to FIG. 2, the precipitation-type negative electrode 400' may include the current collector 401 and a negative electrode coating layer 405 disposed on the current collector. The all-solid-state battery having this precipitation-type negative electrode 400' starts to be initially charged in absence of a negative electrode active material, and a lithium metal with high density and the like are precipitated between the current collector 401 and the negative electrode coating layer 405 during the charge and form a lithium metal layer 404, which may work as a negative electrode active material. Accordingly, the precipitation-type negative electrode 400', in the all-solid-state battery which is more than once charged, may include the current collector 401, the lithium metal layer 404 on the current collector, and the negative electrode coating layer 405 on the lithium metal layer 404. The lithium metal layer 404 means a layer of the lithium metal and the like precipitated during the charge of the battery and may be called to be a metal layer, a negative electrode active material layer, or the like.

The negative electrode coating layer 405 may include metal and/or a carbon material which plays a role of a catalyst.

The metal may include, for example gold, platinum, palladium, silicon silver, aluminum, bismuth, tin, zinc, or a combination thereof and may be composed of one selected therefrom or an alloy of more than one. An average particle diameter (D50) of the metal may be may be less than or equal to 4 µm, for example, 10 nm to 4 µm, 10 nm to 2 µm, or 10 nm to 1 µm.

The carbon material may be, for example, crystalline carbon, non-graphitic carbon, or a combination thereof. For example, the crystalline carbon may be at least one selected from natural graphite, artificial graphite, mesophase carbon microbeads, and a combination thereof. The non-graphitic carbon may be at least one selected from carbon black, activated carbon, acetylene black, denka black, ketjen black, furnace black, graphene, and a combination thereof.

When the negative electrode coating layer 405 includes both the metal and the carbon material, a mixing ratio of the metal and the carbon material may be, for example, a weight ratio of 1:10 to 1:2, 1:10 to 2:1, 5:1 to 1:1, or 4:1 to 2:1. In this case, the precipitation of lithium metal can be effectively promoted and the characteristics of the all-solid-state battery can be improved. For example, the negative electrode coating layer 405 may include a carbon material on which a catalyst metal is supported, or may include a mixture of metal particles and carbon material particles.

The negative electrode coating layer 405 may further include a binder, and the binder may be, for example, a conductive binder. Additionally, the negative electrode coating layer 405 may further include general additives such as fillers, dispersants, and ion conductive materials.

A thickness of the negative electrode coating layer 405 may be for example 1 µm to 20 µm, 2 µm to 10 µm, or 3 µm to 7 µm. Additionally, the thickness of the negative electrode coating layer 405 may be less than or equal to 50%, less than or equal to 20%, or less than or equal to 5% of the thickness of the positive electrode active material layer. If the thickness of the negative electrode coating layer 405 is too thin, it may be collapsed by the lithium metal layer 404, and if the thickness is too thick, the density of the all-solid-state battery may decrease and internal resistance may increase.

The precipitation-type negative electrode 400' may further include a thin film, for example, on the surface of the current collector, that is, between the current collector and the negative electrode catalyst layer. The thin film may include an element capable of forming an alloy with lithium. The element capable of forming an alloy with lithium may be, for example, gold, silver, zinc, tin, indium, silicon, aluminum, bismuth, and the like, which may be used alone or an alloy of more than one. The thin film may further planarize a precipitation shape of the lithium metal layer 404 and much improve characteristics of the all-solid-state rechargeable battery. The thin film may be formed, for example, in a vacuum deposition method, a sputtering method, a plating method, and the like. The thin film may have, for example, a thickness of 1 nm to 800 nm, or 100 nm to 500 nm.

The lithium metal layer 404 may include a lithium metal or a lithium alloy. The lithium alloy may be, for example, a Li-Al alloy, a Li-Sn alloy, a Li-In alloy, a Li-Ag alloy, a Li-Au alloy, a Li-Zn alloy, a Li-Ge alloy, or a Li-Si alloy.

A thickness of the lithium metal layer 404 may be 1 µm to 500 µm, 1 µm to 200 µm, 1µm to 100 µm, or 1 µmm to 50 µm. If the thickness of the lithium metal layer 404 is too thin, it is difficult to perform the role of a lithium storage, and if it is too thick, the battery volume may increase and performance may deteriorate.

When applying such a precipitation-type negative electrode, the negative electrode coating layer 405 may serve to protect the lithium metal layer 404 and suppress the precipitation growth of lithium dendrite. Accordingly, short circuit and capacity degradation of the all-solid-state battery may be suppressed and cycle-life characteristics can be improved.

### [Mode for Invention]

Hereinafter, examples of the present invention and comparative examples are described. It is to be understood, however, that the examples are for the purpose of illustration and are not to be construed as limiting the present invention.

### Synthesis Example 1: Compound Represented by Chemical Formula 1-1a

Compound D (a compound represented by Chemical Formula 1-1a) may be obtained in the method under the following condition 1 or 2.

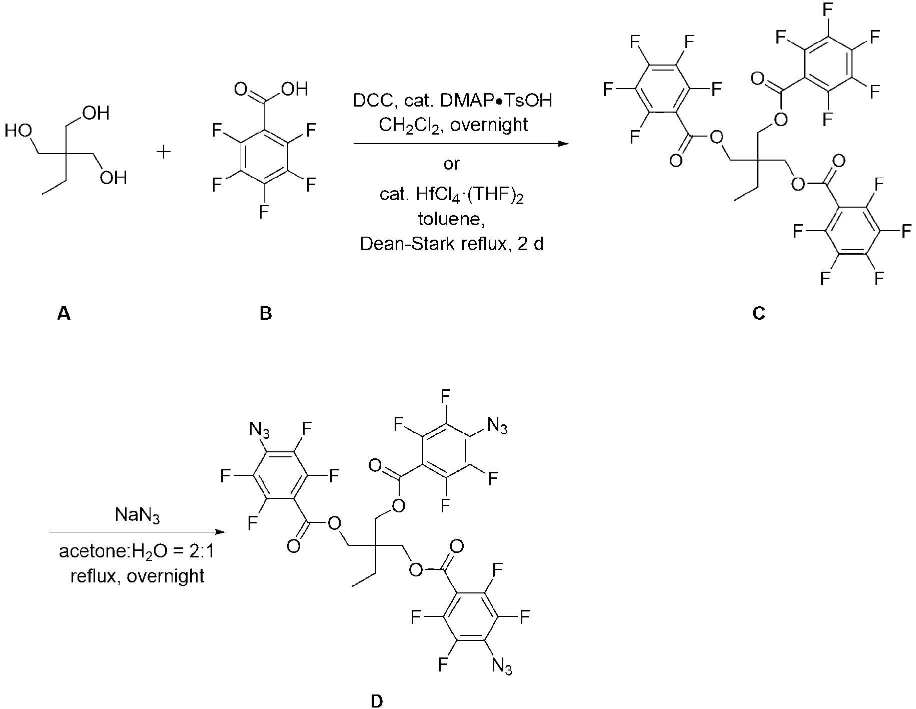

### <Condition 1>

### (1) Step 1: Synthesis Method of Compound C [2-ethyl-2-(((perfluorobenzoyl)oxy)methyl)propane-1,3-diyl bis(2,3,4,5,6-pentafluorobenzoate)]

In a 250 mL round-bottomed flask, trimethylolpropane (Compound **A**) (1.3 g, 9.7 mmol) and pentafluorobenzoic acid (Compound **B**) (10 g, 29 mmol) are mixed and dissolved in 50 mL of dichloromethane (CH₂Cl₂). Subsequently, dicyclohexylcarbodiimide (DCC) (6.0 g, 29 mmol) and 4-dimethylaminopyridinium-paratoluenesulfonate salt (DMAP·TsOH) (0.44 g, 1.5 mmol) are added thereto. The reaction mixture is stirred at room temperature for 24 hours. After checking if a reaction has progressed through TLC, 1 mL of water is added thereto and then, stirred for 10 minutes, and an N,N'-dicyclohexylurea by-product is removed from the reaction mixture by filtration with a filter pater. After adding 100 mL of water to a filtrate therefrom, the mixture is three times extracted with dichloromethane (150 mL x 3 times). The extracted organic layer is collected, washed with salt-saturated water (brine), and dried with anhydrous magnesium sulfate, and a filtrate therefrom is concentrated by using a vacuum rotary evaporator to obtain a white solid. This is recrystallized by using dichloromethane and hexane to obtain Compound **C** as a white solid at a yield of 70%.

### (2) Step 2: Synthesis Method of Compound D [2-(((4-azido-2,3,5,6-tetrafluorobenzoyl)oxy)methyl)-2-ethylpropane-1,3-diyl bis(4-azido-2,3,5,6-tetrafluorobenzoate)]

To a 250 mL brown round-bottomed flask, Compound **C** (2.5 g, 3.5 mmol) and sodium azide (0.91 g, 14 mmol) are added. Subsequently, 30 mL of acetone is added in a dropwise fashion thereto to dissolve Compound **C**, and 15 mL of distilled water is added in a dropwise fashion thereto to dissolve the sodium azide. The reaction mixture is stirred overnight at 50 °C by installing a reflux cooler. After cooling the reaction mixture to room temperature, acetone is removed therefrom by using a rotatary evaporator. Subsequently, water (20 mL) and dichloromethane (50 mL) are added to the reaction mixture, and an aqueous layer is three times extracted with dichloromethane (50 mL x 3 times). The extracted organic layer is washed with salt-saturated water, dried anhydrous magnesium sulfate, and filtered, and a filtrate therefrom is concentrated with a vacuum rotatary evaporator. The concentrated product is recrystallized by using dichloromethane and hexane to obtain desired Compound **D** (a compound represented by Chemical Formula 1-1a) as a white solid at a yield of 77%.

### <Condition 2>

In a 250 mL round-bottomed flask, trimethylolpropane (Compound **A**) (1.9 g, 14 mmol) and pentafluorobenzoic acid (Compound **B**) (17 g, 49 mmol) are mixed and then, dissolved in 150 mL of toluene. Subsequently, a hafnium catalyst (HfCl₄·(THF)₂), 0.23 g, 0.49 mmol) is added thereto. The reaction mixture is stirred for 2 days under heated reflux conditions by installing a Dean-stark trap and a reflux cooler. A reaction proceeds, while removing an azeotropic mixture of toluene and water generated during the reaction. After cooling the reaction mixture to room temperature, a NaHCO₃ solution is added to a filtrate therefrom until it appears basic and then, three times extracted with dichloromethane (150 mL X 3 times). The extracted organic layer is washed with salt-saturated water (brine) and dried with anhydrous magnesium sulfate, and a filtrate therefrom is concentrated with a vacuum rotary evaporator. Subsequently, the concentrated product is purified through column chromatography (hexanes:EtOAc = 4:1, R_{f}=0.63) to obtain Compound **D** (a compound represented by Chemical Formula 1-1a) as a white solid at a yield of 66%. A product therefrom may be purified through column chromatography or by recrystallization in CH₂Cl₂/hexanes.

¹H NMR (CDCl₃, 400 MHz): δ 4.40 (s, 6H), 1.67 (q, J = 7.4 Hz, 2H), 0.99 (t, J = 7.4 Hz, 3H); ¹⁹F NMR (CDCl₃, 376 MHz): δ -138.24 (m, 6F), -150.5 (m, 6F); LRMS(ESI, positive mode): m/z calculated for [M + Na⁺] 808.0533, found 808.1667.

### Synthesis Example 2: Compound Represented by Chemical Formula 1-1b

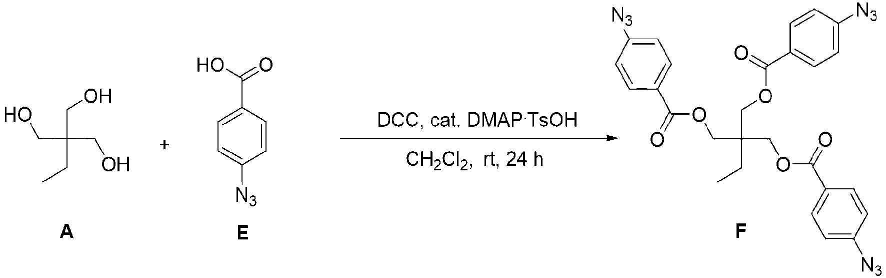

In a 250 ml round-bottomed flask, trimethylolpropane (Compound **A**) (0.44 g, 3.3 mmol) and 4-azidobenzoic acid (Compound **E**) (1.78 g, 10.9 mmol) are mixed and dissolved in 50 mL of dichloromethane (CH₂Cl₂). Subsequently, N,N'-dicyclohexylcarbodiimide (DCC) (2.25 g, 10.9 mmol) and 4-dimethylaminopyridinium-paratoluenesulfonate salt (DMAP·TsOH) (0.16 g, 5 mol%) are added thereto. The reaction mixture is stirred at room temperature for 24 hours. After checking if a reaction is all progressed through TLC, 1 mL of water is added thereto and then, stirred for 10 minutes, and a N,N'-dicyclohexylurea by-product is removed by filtration with a filter paper. After adding 30 mL of water to the filtrate, the mixture is three times extracted with dichloromethane (30 mL x 3 times). The extracted organic layer is gathered, washed with salt-saturated water (brine), dried with anhydrous magnesium sulfate, and filtered, and a filtrate therefrom is concentrated with a vacuum rotary evaporator to obtain a white solid. This is purified through column chromatography (hexanes:EtOAc = 4:1, R_{f}=0.49) to obtain desired compound **F** (a compound represented by Chemical Formula 1-1b) as yellow oil at a yield of 73%.
¹H NMR (CDCl_{3,} 400 MHz): δ 7.98 (d, J = 8.8 Hz, 6H), δ 7.03 (d, J = 8.8 Hz, 6H), δ 4.47 (s, 6H), δ 1.76 (q, J = 7.6 Hz, 2H), δ 1.06 (t, J = 7.6 Hz, 3H)

### Synthesis Example 3: Compound Represented by Chemical Formula 1-2a

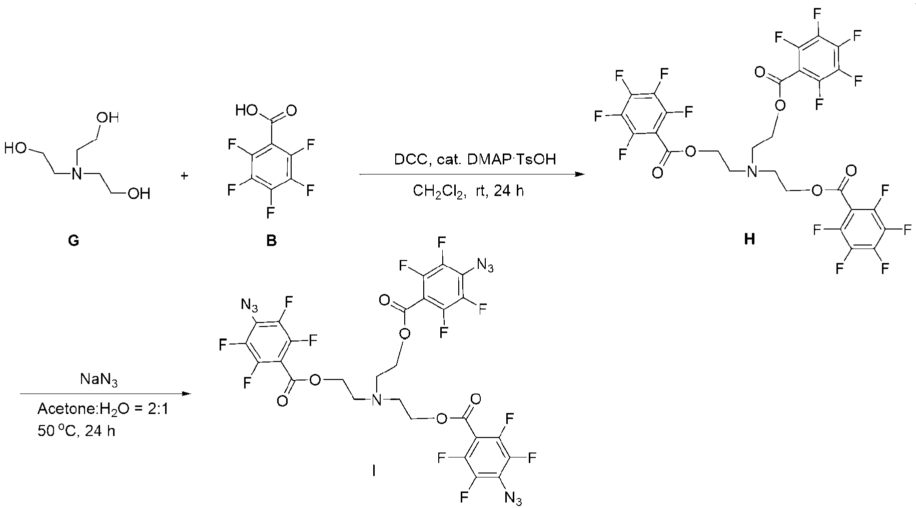

### (1) Synthesis Method of Compound H [nitrilotris(ethane-2,1-diyl) tris(4-azido-2,3,5,6-tetrafluorobenzoate)]

In a 100 mL round-bottomed flask, triethanolamine (Compound **G**) (0.25 g, 1.66 mmol) and pentafluorobenzoic acid (Compound **B**) (1.06 g, 4.99 mmol) are mixed and dissolved in 30 mL of dichloromethane (CH₂Cl₂). Subsequently, N,N'-dicyclohexylcarbodiimide (DCC) (1.03 g, 4.99 mmol) and 4-dimethylaminopyridinium-paratoluenesulfonate salt (DMAP·TsOH) (73 mg, 5 mol%) are added thereto. The reaction mixture is stirred at room temperature for 24 hours. After checking if a reaction has progressed through TLC, 1 mL of water is added thereto and then, stirred for 10 minutes, and an N,N'-dicyclohexylurea by-product is removed therefrom by filtration with a filter paper. Subsequently, 100 mL of water is added to a filtrate therefrom and then, three times extracted with dichloromethane (30 mL x 3 times). The extracted organic layer is washed with salt-saturated water (brine), dried with anhydrous magnesium sulfate, and filtered, and a filtrate therefrom is concentrated by using a vacuum rotary evaporator to obtain Compound **H** as a white solid at a yield of 77%. Since the product is judged to be superior to impurities through ¹H NMR, the next step proceeds without purification. ¹H NMR (CDCl_{3,} 400 MHz): δ 4.44 (t, J = 5.4 Hz, 6H), δ 3.03 (t, J = 5.2 Hz, 6H); ¹⁹F NMR (CDCl_{3,} 376 MHz): δ - 138.32(d, J = 20 Hz, 6H), δ -148.099 (d, J = 22 Hz, 3H), δ -160.27 (d, J = 15.6 Hz, 6H);

### (2) Step 2: Synthesis Method of Compound I [nitrilotris(ethane-2,1-diyl) tris(4-azido-2,3,5,6-tetrafluorobenzoate)]

To a 100 mL round-bottomed flask, Compound **H** (1.21 g, 1.66 mmol) and sodium azide (0.38 g, 5.8 mmol, 3.5 eq) are added. After adding 20 mL of acetone in a dropwise fashion thereto to dissolve Compound **H**, 10 mL of distilled water is added thereto in a dropwise fashion to dissolve the sodium azide. The mixture is stirred at 50 °C for 24 hours by placing a stirring magnet in the flask and installing a reflux cooler thereto. Subsequently, the flask containing the mixture is cooled to room temperature, and the acetone is removed therefrom by using a rotary evaporator. After adding dichloromethane (30 mL) and water (10 mL) to the reaction mixture, and an aqueous layer is three times extracted by dichloromethane (30 mL x 3 times). The extracted organic layer is gathered again and washed with salt-saturated water (brine). Subsequently, a product therefrom is dried with anhydrous magnesium sulfate and filtered to remove anhydrous magnesium sulfate therefrom. A filtrate therefrom is concentrated through a vacuum rotary evaporator to obtain a white solid. A product therefrom is purified through column chromatography (hexanes:EtOAc = 4:1, R_{f}:0.38) to obtain desired Compound **I** (a compound represented by Chemical Formula 1-2a) as a white solid at a yield of 61%.
¹H NMR (400 MHz, CDCl₃): δ 4.43 (t, J = 5.6 Hz, 6H), δ 3.03 (t, J = 5.6 Hz, 6H); ¹⁹F NMR (375 MHz, CDCl₃): δ -138.76 (d, J = 9.6 Hz, 6H), δ -150.95 (d, J = 9.6 Hz, 6H)

### Synthesis Example 4: Compound Represented by Chemical Formula 1-3a

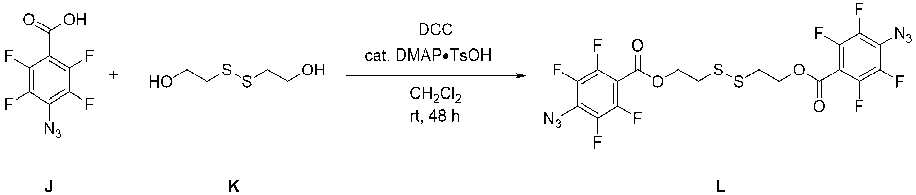

In a 100 mL brown round-bottomed flask, 4-azido-2,3,5,6-tetrafluorobenzoic acid (Compound **J**) (0.9 g, 3.8 mmol) and bis(2-hydroxyethyl)disulfide (Compound **K**) (0.30 g, 1.9 mmol) are mixed and dissolved in 10 mL of dichloromethane (CH₂Cl₂). Subsequently, dicyclohexylcarbodiimide (DCC) (0.79 g, 3.8 mmol) and dimethylaminopyridinium-paratoluenesulfonate salt (DMAP·TsOH) (56 mg, 0.19 mmol) are added thereto. The reaction mixture is stirred at room temperature for 48 hours. After checking if a reaction has progressed through TLC, 0.1 mL of water is added thereto and then, stirred for 10 minutes, and a N,N'-dicyclohexylurea by-product is removed from the reaction mixture by filtration with a filter paper. After adding 10 mL of water to a filtrate therefrom, the mixture is three times extracted with dichloromethane (15 mL x 3 times). The extracted organic layer is washed with salt-saturated water, dried with anhydrous magnesium sulfate, and filtered, and a filtrate therefrom is concentrated with a vacuum rotary evaporator. Subsequently, the concentrated product is purified through column chromatography (hexanes:EtOAc = 4:1, R_{f}=0.55) to obtain Compound **L** (a compound represented by Chemical Formula 1-3a) as a white solid at a yield of 73%.

¹H NMR (CDCl₃, 400 MHz): δ 4.63 (t, J = 6.6 Hz, 4H), 3.66 (t, J = 6.6 Hz, 4H),; ¹⁹F NMR (CDCl₃, 376 MHz): δ -138.24 (m, 6F), -150.77 (m, 6F).

### Synthesis Example 5: Compound Represented by Chemical Formula 1-4a

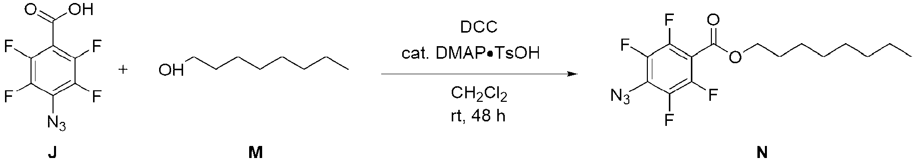

In a 100 mL brown round-bottomed flask, 4-azido-2,3,5,6-tetrafluorobenzoic acid (Compound **J**) (0.80 g, 3.4 mmol) and n-octanol (Compound **M**) (0.44 g, 3.4 mmol) are mixed and dissolved in 5 mL of dichloromethane (CH₂Cl₂). Subsequently, dicyclohexylcarbodiimide (DCC) (0.70 g, 3.4 mmol) and 4-dimethylaminopyridinium-paratoluenesulfonate salt (DMAP·TsOH) (21 mg, 0.17 mmol) are added thereto. The reaction mixture is stirred at room temperature for 48 hours. After checking if a reaction has progressed through TLC, 0.1 mL of water is added thereto and then, stirred for 10 minutes, and a N,N'-dicyclohexylurea by-product is removed from the reaction mixture by filtration with a filter paper. After adding 10 mL of water to a filtrate therefrom, the mixture is three times extracted with dichloromethane (15 mL x 3 times). The extracted organic layer is washed with salt-saturated water, dried with anhydrous magnesium sulfate, and filtered, and a filtrate therefrom is concentrated with a vacuum rotary evaporator. Subsequently, the concentrated product is purified through column chromatography (hexanes:EtOAc = 10:1, R_{f}=0.55) to obtain Compound **N** (a compound represented by Chemical Formula 1-4a) as a white solid at a yield of 78%.

¹H NMR (CDCl₃, 400 MHz): δ 4.36 (t, J = 6.6 Hz, 2H), 1.74 (t, J = 7.6 Hz, 2H), 1.34 (m, 10H), 0.88 (t, J = 5.9 Hz, 2H).; ¹⁹F NMR (CDCl₃, 376 MHz): δ - 138.84 (m, 6F), -150.97 (m, 6F).

### Comparative Synthesis Example 1: Compound Represented by Chemical Formula 1-1c

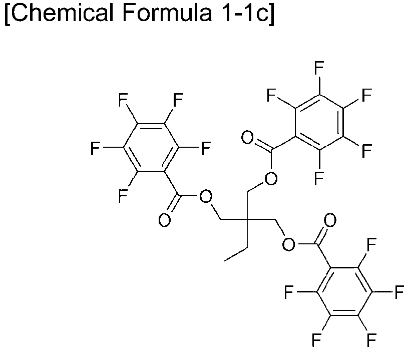

In a 250 mL round-bottomed flask, trimethylolpropane (Compound **A**) (1.3 g, 9.7 mmol) and pentafluorobenzoic acid (Compound **B**) (10 g, 29 mmol) are mixed and dissolved in 50 mL of dichloromethane (CH₂Cl₂). Subsequently, dicyclohexylcarbodiimide (DCC) (6.0 g, 29 mmol) and dimethylaminopyridinium-paratoluenesulfonate salt (DMAP·TsOH) (0.44 g, 1.5 mmol) were added thereto. The reaction mixture is stirred at room temperature for 24 hours. After checking if a reaction has progressed through TLC, 1 mL of water is added thereto and then, stirred for 10 minutes, and a N,N'-dicyclohexylurea by-product is removed from the reaction mixture by filtration with a filter paper. After adding 10 mL of water to a filtrate therefrom, the mixture is three times extracted with dichloromethane (15 mL x 3 times). The extracted organic layer is washed with salt-saturated water, dried with anhydrous magnesium sulfate, and filtered, and a filtrate therefrom is concentrated with a vacuum rotary evaporator to obtain a white solid. This is recrystallized with dichloromethane and hexane to obtain Compound **C** (a compound represented by Chemical Formula 1-1c) as a white solid at a yield of 70%.

### Example 1

### (1) Preparation of Solid Electrolyte Layer

An acryl-based binder (SX-A334, Zeon Corp.) is dissolved in an isobutyryl isobutyrate (octyl acetate, OA) solvent to prepare a binder solution, and an argyrodite-type solid electrolyte Li₆PS₅Cl (D50 = 3 µm) and the additive of Synthesis Example 1 (a compound represented by Chemical Formula 1-1a) in a weight ratio of 95:5 are added thereto and then, stirred in a Thinky mixer to secure appropriate viscosity. After adjusting the viscosity, 2 mm zirconia balls are added thereto and then, stirred again with the Thinky mixer to prepare slurry. The slurry includes 93.1 wt% of the solid electrolyte, 4.9 wt% of the additive of Synthesis Example 1, and 2 wt% of the binder. The slurry is applied on a release PET film with a bar coater and dried at room temperature to form a solid electrolyte layer.

### (2) Manufacture of Positive Electrode

85 wt% of a positive electrode active material, LiNi_{0.9}Co_{0.05}Mn_{0.05}O₂, 13.5 wt% of a lithium argyrodite-type solid electrolyte, Li₆PS₅Cl, 1.0 wt% of a polyvinylidene fluoride binder, and 0.5 wt% of a carbon nanotube conductive material are prepared and then, added to a dispersive medium prepared by mixing octyl acetate (OA) and pentyl propionate (PPP) in a weight ratio of 1:1. The obtained mixture is added to a Thinky mixer, and 2 mm zirconia balls are added thereto and then, stirred to prepare a positive electrode composition. A content of the dispersive medium is 30 parts by weight based on 100 parts by weight of a solid content. The solid content is a total of the positive electrode active material, the solid electrolyte, the binder, and the conductive material.

The prepared positive electrode composition is coated on a positive electrode current collector with a bar coater and dried at 80 °C for 10 minutes in a convention oven to form a positive electrode active material layer thereon, manufacturing a positive electrode having positive electrode active material layer on the current collector.

### (3) Manufacture of Negative Electrode

After preparing a catalyst by mixing carbon black with a primary particle diameter (D50) of about 30 nm and silver (Ag) with an average particle diameter (D50) of about 60 nm in a weight ratio of 3:1, 0.25 g of the catalyst is added to 2 g of an NMP solution including 7 wt% of a polyvinylidene fluoride binder and then, mixed, preparing a negative electrode coating layer composition. This is coated on a nickel foil current collector using a bar coater and dried in vacuum to prepare a precipitated negative electrode with a negative electrode coating layer formed on the current collector.

### (4) Manufacture of All-solid-state Battery Cell

After cutting the positive electrode, negative electrode, and solid electrolyte layer, the solid electrolyte layer is stacked on the positive electrode, and the negative electrode is stacked thereon. This is sealed in the form of a pouch and then, subjected to warm Isostatic press (WIP) at a high temperature of 85 °C under 500 MPa for 30 minutes to manufacture an all-solid-state battery cell. In this pressurized state, the positive electrode active material layer has a thickness of about 100 µm, the negative electrode coating layer has a thickness of about 7 µm, and the solid electrolyte layer has a thickness of about 60 µm.

### Example 2

A solid electrolyte layer and an all-solid-state battery cell are manufactured in the same method as in Example 1 except that the additive of Synthesis Example 2 (a compound represented by Chemical Formula 1-1b) is used instead of the additive of Synthesis Example 1 (a compound represented by Chemical Formula 1-1a).

### Example 3

A solid electrolyte layer and an all-solid-state battery cell are manufactured in the same method as in Example 1 except that the additive of Synthesis Example 3 (a compound represented by Chemical Formula 1-2a) is used instead of the additive of Synthesis Example 1 (a compound represented by Chemical Formula 1-1a).

### Example 4

A solid electrolyte layer and an all-solid-state battery cell are manufactured in the same method as in Example 1 except that the additive of Synthesis Example 4 (a compound represented by Chemical Formula 1-3a) is used instead of the additive of Synthesis Example 1 (a compound represented by Chemical Formula 1-1a).

### Example 5

A solid electrolyte layer and an all-solid-state battery cell are manufactured in the same method as in Example 1 except that the additive of Synthesis Example 5 (a compound represented by Chemical Formula 1-4a) is used instead of the additive of Synthesis Example 1 (a compound represented by Chemical Formula 1-1a).

### Example 6

A solid electrolyte layer is formed by using the argyrodite-type solid electrolyte Li₆PS₅Cl (D50 = 3 µm) and the additive of Synthesis Example 1 in a weight ratio of 97:3 to prepare slurry including 95.06 wt% of the solid electrolyte, 2.94 wt% of the additive of Synthesis Example 1, and 2 wt% of the binder. An all-solid-state battery cell is manufactured in the same manner as in Example 1 except for this.

### Example 7

A solid electrolyte layer is formed by using the argyrodite-type solid electrolyte Li₆PS₅Cl (D50 = 3 µm) and the additive of Synthesis Example 1 in a weight ratio of 90:10 to prepare slurry including 88.2 wt% of the solid electrolyte, 9.8 wt% of the additive of Synthesis Example 1, and 2 wt% of the binder. An all-solid-state battery cell is manufactured in the same manner as in Example 1 except for this.

### Comparative Example 1 (Ref.)

A solid electrolyte layer is formed by adding the argyrodite-type solid electrolyte Li₆PS₅Cl (D50 = 3 µm) alone to prepare slurry including 98 wt% of the solid electrolyte and 2 wt% of the binder. An all-solid-state battery cell is manufactured in the same manner as in Example 1 except for this.

### Evaluation Example 1: Thermogravimetric Analysis (TGA)

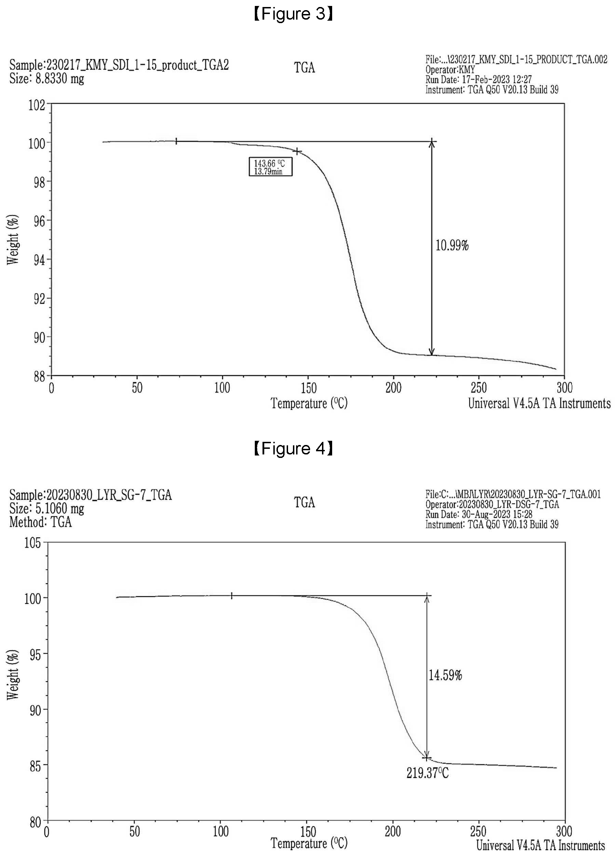

A thermogravimetric analysis of the additives of Synthesis Examples 1 to 4 and Comparative Synthesis Example 1 is performed within a temperature range of 30 to 300 °C, and the results are shown in FIG. 3.

The thermogravimetric analysis is performed by taking about 10 mg of the additive and using TGA8000 made by PerkinElmer Inc.

FIG. 3 is a TGA thermal analysis diagram of the additive (the compound represented by Chemical Formula 1-1a) of Synthesis Example 1. Referring to FIG. 3, the compound represented by Chemical Formula 1-1a exhibits a weight loss of about 11% from about 140 °C to 230 °C.

FIG. 4 is a TGA thermal analysis diagram of the additive (the compound represented by Formula 1-1b) of Synthesis Example 2. Referring to FIG. 4, the compound represented by Chemical Formula 1-1b exhibits a weight loss of about 15% from about 140 °C to 220 °C.

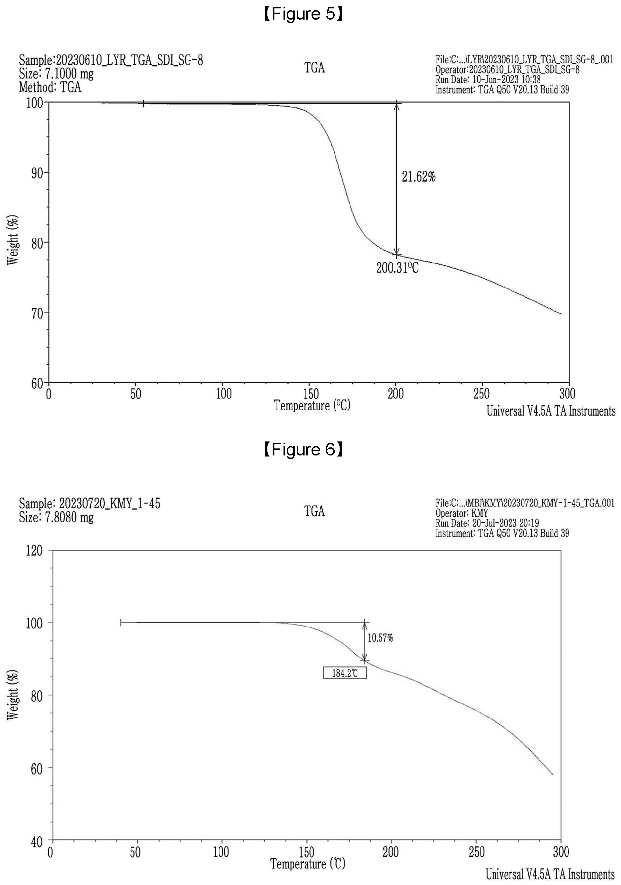

FIG. 5 is a TGA thermal analysis diagram of the additive (the compound represented by Chemical Formula 1-2a) of Synthesis Example 3. Referring to FIG. 5, the compound represented by Chemical Formula 1-2a exhibits a weight loss of about 22% from about 140 °C to 220 °C.

FIG. 6 is a TGA thermal analysis diagram of the additive (the compound represented by Chemical Formula 1-3a) of Synthesis Example 4. Referring to FIG. 6, the compound represented by Chemical Formula 1-3a exhibits a weight loss of about 11% from about 140 °C to 185 °C.

During the TGA analysis of each of the additives of Synthesis Examples 1 to 4, the results are similar to a theoretically calculated weight loss amount after generating nitrogen gas.

Accordingly, an azide group (azide, -N₃) in each of the additive of Synthesis Examples 1 to 4 is decomposed at a high temperature of about 140 °C or higher and generates nitrogen (N₂) and then, converted to a nitrene group (nitrene, -N).

### Evaluation Example 2: Differential Scanning Calorimetry (DSC)

Each of the all-solid-state battery cells of Examples 1 to 5 and Comparative Example 1 is analyzed within a temperature range of 40 to 200 °C through differential scanning calorimetry (DSC).

After charging each of the all-solid-state battery cells to 4.20 V, a sample for DSC is prepared in a glove box under an Ar atmosphere according to the following steps. A single cell is taken out from a laminate bag of an exterior body and drilled out to a hole with φ 2.5 mm with a mold. The sample is placed in a sample pan made of SUS, covered with a lid, and joined to seal the entrance with a press. This prepared sample for DSC is measured at a temperature range of 40 to 200 °C by using a DSC measuring device (DSC7000X) made by Hitachi High-Tech Science.

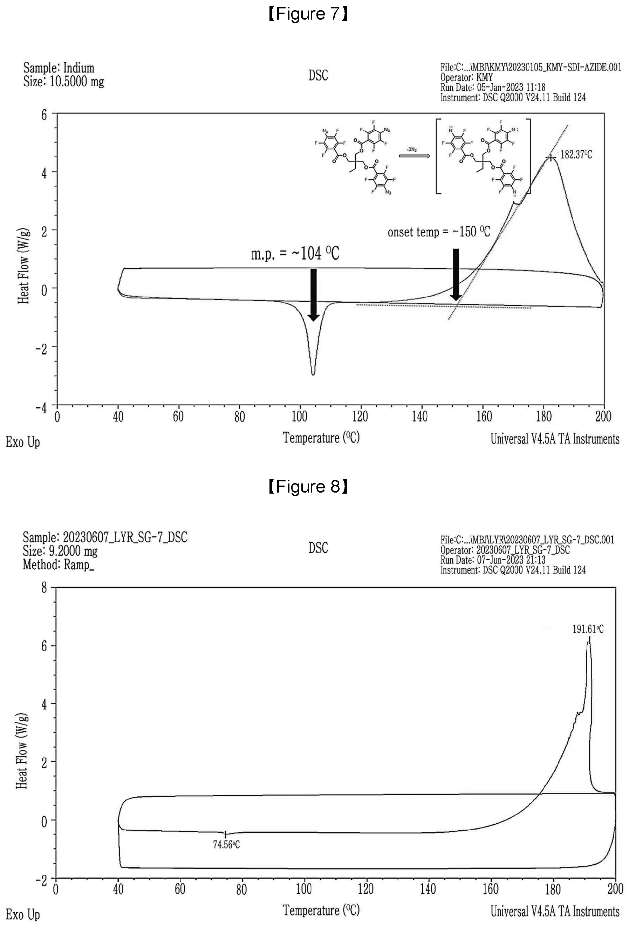

FIG. 7 is a DSC analysis diagram of the all-solid-state battery cell of Example 1 to which the additive (compound represented by Chemical Formula 1-1a) of Synthesis Example 1 is applied.

FIG. 8 is a DSC analysis diagram of the all-solid-state battery cell of Example 1 to which the additive of Synthesis Example 2 (compound represented by Chemical Formula 1-1b) is applied.

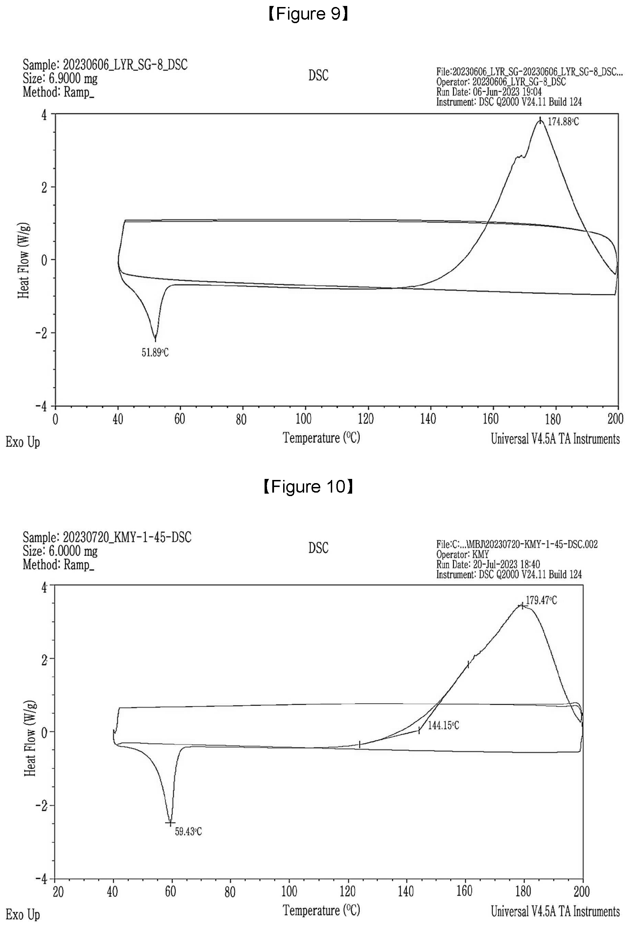

FIG. 9 is a DSC analysis diagram of the all-solid-state battery cell of Example 1 to which the additive (compound represented by Chemical Formula 1-2a) of Synthesis Example 3 is applied.

FIG. 10 is a DSC analysis diagram of the all-solid-state battery cell of Example 1 to which the additive (compound represented by Chemical Formula 1-3a) of Synthesis Example 4 is applied.

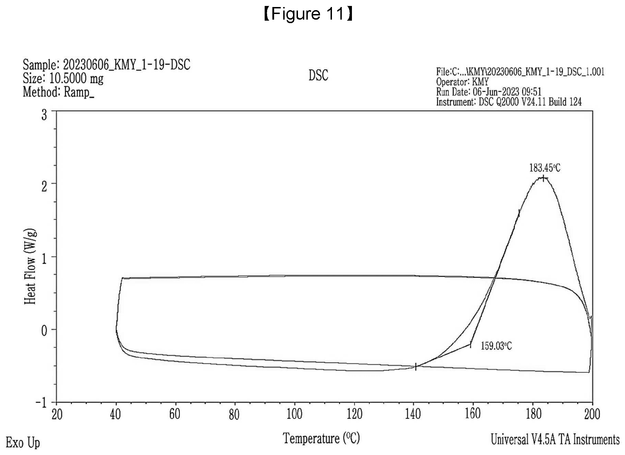

FIG. 11 is a DSC analysis diagram of the all-solid-state battery cell of Example 1 to which the additive of Synthesis Example 5 (compound represented by Chemical Formula 1-4a) is applied.

In the solid electrolyte layers of Examples 1 to 5, an azide group (azide, - N₃) in each additive is decomposed to generate nitrogen (N₂) and converted to a nitrene group (nitrene, -N) at a high temperature of 140 °C or higher during the first cycle.

### Evaluation Example 3: Lithium Ionic Conductivity Analysis

Each of the solid electrolyte layers of Examples 1 to 5 and Comparative Example 1 is heated at 180 °C for 30 minutes and then, measured with respect to ionic conductivity changes, and the results are shown in Table 1.

The ionic conductivity is measured through electrochemical impedance spectroscopy (EIS), wherein EIS is performed at an amplitude of about 10 mV and a frequency of 0.1 to 0.05 Hz at 0.1 MHz under an air atmosphere at 25 °C.

**(Table 1)**

| | Ionic conductivity (mS/cm) | | Retention rate of Ionic conductivity (%) |
|---|---|---|---|
| | Less than 180 °C | 180 °C or more | |
| Example 1 | 0.68 | - | - |
| Example 2 | 0.67 | - | - |
| Example 3 | 0.68 | - | - |
| Example 4 | 0.69 | - | - |
| Example 5 | 0.67 | - | - |
| Comparative Example 1 | 0.70 | 0.67 | 95.7 |

| | | | |
|---|---|---|---|
| (In Table 1, '-' means that the ionic conductivity is too low to be measurable, as the additive effect effectively shut down the system at 180 °C or higher.) | | | |

Referring to Table 1, the additive of an embodiment represented by Examples 1 to 5 sharply reduces ionic conductivity at a high temperature of 180 °C or higher and thus has an effect of shutting down ignition of the rechargeable lithium battery cells at a lower temperature than the ignition temperature.

Although the preferred embodiments have been described in detail above, the scope of the present invention is not limited thereto. In addition, various modifications and improvements made by those skilled in the art using the basic concept defined in the claims should also be understood as falling within the scope of the present invention.

### <Description of Symbols>

| | | | |
|---|---|---|---|
| 100: | all-solid-state battery | 200: | positive electrode |
| 201: | positive electrode current collector | | |
| 203: | positive electrode active material layer | | |
| 300: | solid electrolyte layer | 400: | negative electrode |
| 401: | negative current collector | | |
| 403: | negative electrode active material layer | | |
| 400': | precipitation-type negative electrode | 404: | lithium metal layer |
| 405: | negative electrode coating layer | 500: | elastic layer |

## Claims

1. An additive for a rechargeable lithium battery represented by Chemical Formula 1: 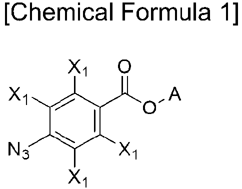
wherein, in Chemical Formula 1,
X₁ is the same or different from each other and is hydrogen or a halogen atom; and
A is a substituent represented by Chemical Formula 2, a substituent represented by Chemical Formula 3, a substituent represented by Chemical Formula 4, a substituent represented by Chemical Formula 5, or a substituted or unsubstituted C1 to C20 alkyl group; 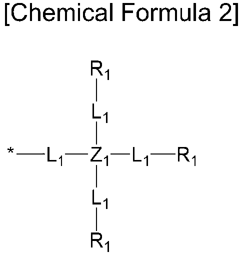
wherein, in Chemical Formula 2,
Z₁ is a carbon atom or a silicon atom,
L₁ is the same or different from each other and is a single bond or a substituted or unsubstituted C1 to C20 alkylene group; and
R₁ is the same or different from each other and is hydrogen, a substituted or unsubstituted C1 to C20 alkyl group, or a substituent represented by Chemical Formula 5; 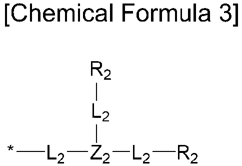
wherein, in Chemical Formula 3,
Z₂ is a nitrogen atom or a phosphorus atom;
L₂ is the same or different from each other and a single bond or a substituted or unsubstituted C1 to C20 alkylene group; and
R₂ is a hydrogen atom, a substituted or unsubstituted C1 to C20 alkyl group, or a substituent represented by Chemical Formula 5;
[Chemical Formula 4] *-L₃-R₃
wherein, in Chemical Formula 4, L3 is the same or different from each other and is a single bond, a disulfide bond, a substituted or unsubstituted C1 to C20 alkylene group, or a combination thereof; and
R₃ is a hydrogen atom, a substituted or unsubstituted C1 to C20 alkyl group, or a substituent represented by Chemical Formula 5; 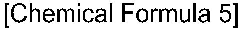 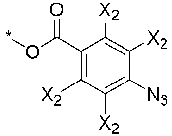
wherein, in Chemical Formula 5,
X₂ is the same or different from each other and is hydrogen or a halogen atom.

2. The additive for a rechargeable lithium battery as claimed in claim 1, wherein
Chemical Formula 1 is represented by any one of Chemical Formulas 1-1 to 1-4: 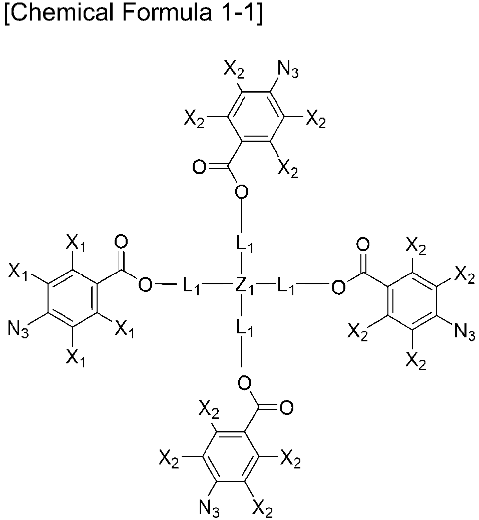 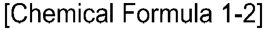 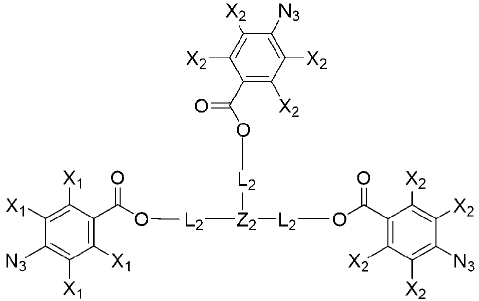 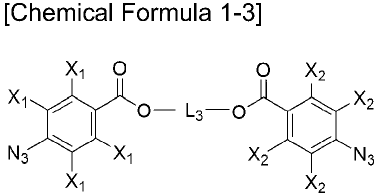 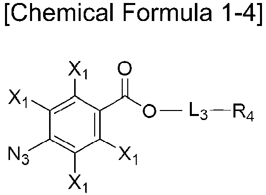
wherein, in Chemical Formulas 1-1 to 1-4,
Z₁ is a carbon atom or a silicon atom;
Z₂ is a nitrogen atom or a phosphorus atom;
X₁ is the same or different from each other and is hydrogen or a halogen atom;
X₂ is the same or different from each other and is hydrogen or a halogen atom;
L₁ is the same or different from each other and is a single bond or a substituted or unsubstituted C1 to C20 alkylene group;
L₂ is the same or different from each other and a single bond or a substituted or unsubstituted C1 to C20 alkylene group;
L₃ is the same or different from each other and is a single bond, a disulfide bond, a substituted or unsubstituted C1 to C20 alkylene group, or a combination thereof; and
R₄ is a hydrogen atom or a substituted or unsubstituted C1 to C20 alkyl group.

3. The additive for a rechargeable lithium battery as claimed in claim 1, wherein
X₁s are all hydrogen atoms or all halogen atoms.

4. The additive for a rechargeable lithium battery as claimed in claim 1, wherein
Z₁ is a carbon atom.

5. The additive for a rechargeable lithium battery as claimed in claim 1, wherein
L₁s are all methylene groups.

6. The additive for a rechargeable lithium battery as claimed in claim 1, wherein
L₂s are all ethylene groups.

7. The additive for a rechargeable lithium battery as claimed in claim 1, wherein
Z₂ is a nitrogen atom.

8. The additive for a rechargeable lithium battery as claimed in claim 1, wherein
L₃ is a single bond; or a combination of a disulfide bond and an ethylene group.

9. The additive for a rechargeable lithium battery as claimed in claim 1, wherein
X₂s are all hydrogen atoms or all halogen atoms.

10. The additive for a rechargeable lithium battery as claimed in claim 1, wherein
the additive is any one selected from the following group:

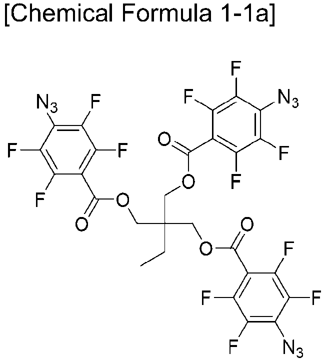

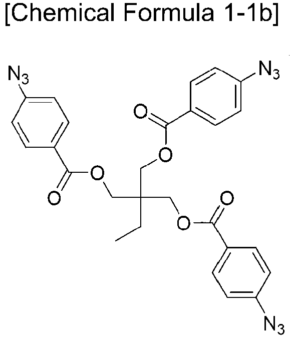

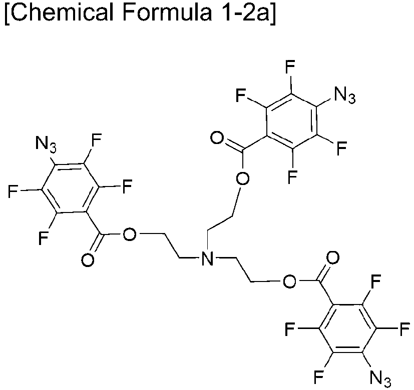

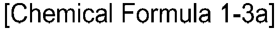

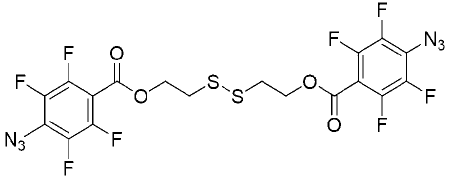

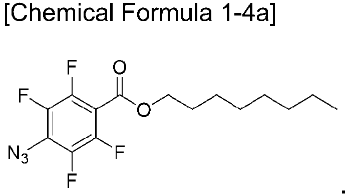

11. An electrolyte layer for a rechargeable lithium battery, comprising
the additive as claimed in any one of claims 1 to 10.

12. The electrolyte layer for a rechargeable lithium battery as claimed in claim 11, wherein
based on a total amount of 100 wt% of the electrolyte layer, the additive is included in an amount of 0.1 to 10 wt%, or 0.1 to 5 wt%.

13. The electrolyte layer for a rechargeable lithium battery as claimed in claim 11, wherein
the electrolyte layer further includes a solid electrolyte.

14. The electrolyte layer for a rechargeable lithium battery as claimed in claim 13, wherein
a weight ratio of solid electrolyte : additive in the electrolyte layer is 99.9:0.01 to 90:10.

15. A positive electrode for a rechargeable lithium battery, comprising
the additive as claimed in any one of claims 1 to 10.

16. A negative electrode for a rechargeable lithium battery, comprising
the additive as claimed in any one of claims 1 to 10.

17. A rechargeable lithium battery, comprising
a positive electrode;
a negative electrode; and
an electrolyte layer between the positive electrode and the negative electrode,
wherein at least one layer of the positive electrode, the negative electrode, and the electrolyte layer includes the additive of any one as claimed in claim 1 to claim 10.

18. The rechargeable lithium battery as claimed in claim 17, wherein
the rechargeable lithium battery is an all-solid-state battery, a semi-solid battery, a lithium metal battery, or a lithium ion battery.
